(19) Europäisches Patentamt
European Patent Office
Office européen des brevets

(11) **EP 3 502 268 B1**

(12) **EUROPEAN PATENT SPECIFICATION**

(45) Date of publication and mention
of the grant of the patent:
**29.01.2020 Bulletin 2020/05**

(51) Int Cl.:
**C12Q 1/04** *(2006.01)*          **C12Q 1/37** *(2006.01)*
**G01N 33/52** *(2006.01)*

(21) Application number: **18214972.4**

(22) Date of filing: **20.12.2018**

(54) **TEST STRIP FOR MICROORGANISM DETECTION AND DETECTION METHOD USING THE SAME**

TESTSTREIFEN ZUM NACHWEIS VON MIKROORGANISMEN UND NACHWEISVERFAHREN DAMIT

BANDE DE TEST POUR LA DÉTECTION DE MICRO-ORGANISMES ET PROCÉDÉ DE DÉTECTION L'UTILISANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.12.2017 US 201762608177 P**

(43) Date of publication of application:
**26.06.2019 Bulletin 2019/26**

(73) Proprietor: **Industrial Technology Research Institute**
**31040 Hsinchu (TW)**

(72) Inventors:
• **Liu, Tseng-Huang**
  **825 Kaohsiung City (TW)**
• **Tang, Chia-Ying**
  **300 Hsinchu City (TW)**
• **Jiang, Pei-Shin**
  **300 Hsinchu City (TW)**

(74) Representative: **Michalski Hüttermann & Partner Patentanwälte mbB**
**Speditionstraße 21**
**40221 Düsseldorf (DE)**

(56) References cited:
**WO-A1-2008/119181     WO-A1-2015/183659**
**US-A1- 2016 273 055**

• **ZHAO ET AL: "Electrospun fibrous mats with conjugated tetraphenylethylene and mannose for sensitive on fluorescent sensing of Escherichia coli", ACS APPL. MATER. INTERFACES,, vol. 7, 18 February 2015 (2015-02-18), pages 5177-5186, XP002777203,**
• **KARIMZADEH AYUB ET AL: "Peptide based biosensors", TRAC TRENDS IN ANALYTICAL CHEMISTRY, ELSEVIER, AMSTERDAM, NL, vol. 107, 1 August 2018 (2018-08-01), pages 1-20, XP085478503, ISSN: 0165-9936, DOI: 10.1016/J.TRAC.2018.07.018**

## Description

### TECHNICAL FIELD

The present disclosure is related to detection of microorganisms, and it is particularly related to a test strip for microorganism detection and a microorganism detection method.

### BACKGROUND

Traditional microbial detection methods need to be carried out through culturing, biochemical detection, microscopic examination, etc., which are time-consuming and laborious. Moreover, the test samples require pre-treatment steps, but the steps are cumbersome and it is not easy to directly and immediately monitor the state of microorganisms in the environment. L. Zhao et al: ACS APPLIED MATERERIALS & INTERFACES, vol. 7, 18 February 2015 pages 5177-5186 discloses electrospun fibrous mats with conjugated tetraphenylethylene and mannose for sensitive turn-on fluorescent sensing of *Escherichia coli.*

Instruments such as biosensors have been developed to perform real-time monitoring and detection of food microorganisms, and they are used widely, especially to maintain quality control of food. However, the use of such instruments and equipment is restricted to the inspection of random samples, as it is impossible to fully inspect the quality of all products. In addition, instruments and equipment cannot be popularized in practical application due to its high cost.

Therefore, it is extremely necessary to develop a simple, effective and low-cost microbial detection method.

### SUMMARY

The present disclosure provides a test strip for microorganism detection, comprising: a porous substrate, a surface of which has a first color; and a microorganism detection composition which is at least adhered to a part of a surface of the porous substrate or at least adhered to a part of a surface of the porous substrate and a part of an interior of the porous substrate. The microorganism detection composition comprises: a plurality of colored particles having a second color that is different than the first color; and a plurality of specific peptides immobilized on the surfaces of the plurality of colored particles, in which the plurality of specific peptides connect the plurality of colored particles to each other to make the plurality of colored particles aggregate to cover the part of the surface of the porous substrate and present the second color on the part of the surface of the porous substrate. Moreover, the specific peptide is designed for a specific microorganism, and the specific microorganism has the ability to cleave the specific peptide.

The present disclosure also provides a microorganism detection method, comprising bringing a test sample into contact with the test strip for microorganism detection mentioned above; and determining the color change of the test strip for microorganism detection after a test sample comes into contact with the test strip for microorganism detection is completed. When the test sample contains the specific microorganism, the specific microorganism cleaves the specific peptide, and that leads to the plurality of colored particles no longer aggregating to lighten the second color, which is present due to the aggregation of the plurality of colored particles, or to expose part of the surface of the porous substrate, thereby making the first color visible.

A detailed description is given in the following embodiments with reference to the accompanying drawings.

### BRIEF DESCRIPTION OF DRAWINGS

The present invention can be more fully understood by reading the subsequent detailed description and examples with references made to the accompanying drawings, wherein:

FIG. 1A shows a top view of the test strip for microorganism detection 100 of the present disclosure;
FIG. 1B show a sectional view of the structure of the test strip for microorganism detection 100 in one embodiment of the present disclosure;
FIG. 1C show a sectional view of the structure of the test strip for microorganism detection 100 in another embodiment of the present disclosure;
FIG. 2 shows a schematic diagram of the plurality of colored particles being connected to each other by the plurality of specific peptides in the microorganism detection composition;
FIG. 3 is a schematic diagram showing one embodiment of a mode for forming the test strip for microorganism detection 100 of the present disclosure shown in FIG. 1B;
FIGs. 4A-4C are schematic diagrams showing different embodiments of a mode for forming the test strip for microorganism detection 100 of the present disclosure shown in FIG. 1C;

FIG. 5 shows a schematic diagram of an usage scenario for detecting specific microorganism in a test sample by the microorganism detection method of the present disclosure;

FIG. 6 show the results of dropping the magnetic bead suspension on the photocopy paper, the filter paper, the nitrocellulose film and the PVDF film;

FIG. 7 show the results of analyzing the test strips respectively obtained by the two methods recited in Example 3 by a scanning electron microscope;

FIG. 8A shows photographs of the test strip treated with proteinase K with different dilution ratios in an enzymatic degradation test, in which the test strip contains magnetic beads on which $\alpha$1-23-mer peptide is immobilized;

FIG. 8B shows the digestion levels (DL) of peptide of the test strip treated with proteinase K with different dilution ratios in an enzymatic degradation test, in which the test strip contains magnetic beads on which $\alpha$1-23-mer peptide is immobilized;

FIG. 9A shows photographs of the test strip treated with trypsin with different dilution ratios in an enzymatic degradation test, in which the test strip contains magnetic beads on which $\beta$2-23-mer peptide is immobilized;

FIG. 9B shows the digestion levels of peptide of the test strip treated with trypsin with different dilution ratios in an enzymatic degradation test, in which the test strip contains magnetic beads on which $\beta$2-23-mer peptide is immobilized;

FIG. 10A shows photographs of the test strip treated with different lactic acid bacteria in a lactic acid bacteria test, in which the test strip contains magnetic beads on which $\alpha$1-23-mer peptide is immobilized;

FIG. 10B shows the detachment levels of the test strip treated with different lactic acid bacteria in a lactic acid bacteria test, in which the test strip contains magnetic beads on which $\alpha$1-23-mer peptide without modification is immobilized;

FIG. 11A shows photographs of the test strip treated with different lactic acid bacteria in a lactic acid bacteria test, in which the test strip contains magnetic beads on which $\beta$2-23-mer peptide is immobilized;

FIG. 11B shows the detachment levels of the test strip treated with different lactic acid bacteria in a lactic acid bacteria test, in which the test strip contains magnetic beads on which $\beta$2-23-mer peptide without modification is immobilized;

FIG. 12A shows photographs of the test strip treated with proteinase K with different dilution ratios in an enzymatic degradation test, in which the test strip is prepared by item 3.1.2 in Method A of Example 3 and contains magnetic beads on which $\alpha$1-23-mer-NH$_2$ peptide is immobilized;

FIG. 12B shows the digestion levels of peptide of the test strip treated with proteinase K with different dilution ratios in an enzymatic degradation test, in which the test strip is prepared by item 3.1.2 in Method A of Example 3 and contains magnetic beads on which $\alpha$1-23-mer-NH$_2$ peptide is immobilized;

FIG. 12C shows the area changes of the region covered by the magnetic beads of the test strip treated with proteinase K with different dilution ratios in an enzymatic degradation test, in which the test strip is prepared by item 3.1.2 in Method A of Example 3 and contains magnetic beads on which $\alpha$1-23-mer-NH$_2$ peptide is immobilized;

FIG. 12D shows the detachment levels of the test strip treated with proteinase K with different dilution ratios in an enzymatic degradation test, in which the test strip is prepared by item 3.1.2 in Method A of Example 3 and contains magnetic beads on which $\alpha$1-23-mer-NH$_2$ peptide is immobilized;

FIG. 13A shows photographs of the test strip treated with trypsin with different dilution ratios in an enzymatic degradation test, in which the test strip is prepared by item 3.1.2 in Method A of Example 3 and contains magnetic beads on which $\beta$2-23-mer-NH$_2$ peptide is immobilized;

FIG. 13B shows the digestion levels of peptide of the test strip treated with trypsin with different dilution ratios in an enzymatic degradation test, in which the test strip is prepared by item 3.1.2 in Method A of Example 3 and contains magnetic beads on which $\beta$2-23-mer-NH$_2$ peptide is immobilized;

FIG. 13C shows the area changes of the region covered by the magnetic beads of the test strip treated with trypsin with different dilution ratios in an enzymatic degradation test, in which the test strip is prepared by item 3.1.2 in Method A of Example 3 and contains magnetic beads on which $\beta$2-23-mer-NH$_2$ peptide is immobilized;

FIG. 13D shows the detachment levels of the test strip treated with trypsin with different dilution ratios in an enzymatic degradation test, in which the test strip is prepared by item 3.1.2 in Method A of Example 3 and contains magnetic beads on which $\beta$2-23-mer-NH$_2$ peptide is immobilized;

FIG. 14 shows photographs of the test strip treated with proteinase K with different dilution ratios in an enzymatic degradation test, in which the test strip is prepared by Method B of Example 3 and contains magnetic beads on which $\alpha$1-23-mer-NH$_2$ peptide is immobilized;

FIG. 15 shows photographs of the test strip treated with trypsin with different dilution ratios in an enzymatic degradation test, in which the test strip is prepared by Method B of Example 3 and contains magnetic beads on which $\beta$2-23-mer-NH$_2$ peptide is immobilized;

FIG. 16A shows photographs of the test strip treated with proteinase K with different dilution ratios in an enzymatic degradation test, in which the test strip is prepared by item 3.1.2 in Method A of Example 3 and contains magnetic

beads on which $\alpha$1-35-mer-NH$_2$ peptide is immobilized;

FIG. 16B shows the detachment levels of the test strip treated with proteinase K with different dilution ratios in an enzymatic degradation test, in which the test strip is prepared by item 3.1.2 in Method A of Example 3 and contains magnetic beads on which $\alpha$1-35-mer-NH$_2$ peptide is immobilized;

FIG. 17A shows photographs of the test strip treated with trypsin with different dilution ratios in an enzymatic degradation test, in which the test strip is prepared by item 3.1.2 in Method A of Example 3 and contains magnetic beads on which $\beta$2-35-mer-NH$_2$ peptide is immobilized;

FIG. 17B shows the detachment levels of the test strip treated with trypsin with different dilution ratios in an enzymatic degradation test, in which the test strip is prepared by item 3.1.2 in Method A of Example 3 and contains magnetic beads on which $\beta$2-35-mer-NH$_2$ peptide is immobilized;

FIG. 18A shows photographs of the test strip containing magnetic beads on which $\alpha$1-23-mer-NH$_2$ peptide or $\beta$2-23-mer-NH$_2$ peptide is immobilized in a lactic acid bacteria test at a low temperature;

FIG. 18B shows the detachment levels of the test strip containing magnetic beads on which $\alpha$1-23-mer-NH$_2$ peptide is immobilized in a lactic acid bacteria test at a low temperature;

FIG. 18C shows the detachment levels of the test strip containing magnetic beads on which $\beta$2-23-mer-NH$_2$ peptide is immobilized in a lactic acid bacteria test at a low temperature;

FIG. 19A shows photographs of the test strip containing magnetic beads on which $\alpha$1-23-mer-NH$_2$ peptide is immobilized in a confirmation test for the minimum detectable amount of *Lactobacillus plantarum* for the test strip; and

FIG. 19B shows the detachment levels of the test strip containing magnetic beads on which $\alpha$1-23-mer-NH$_2$ peptide is immobilized in a confirmation test for the minimum detectable amount of *Lactobacillus plantarum* for the test strip.

## DETAILED DESCRIPTION

In the following detailed description, for purposes of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

The present disclosure provides a test strip for microorganism detection which is light and thin, and is easy to carry and use. The test strip for microorganism detection of the present disclosure is used for detecting the presence of a specific microorganism. By using the test strip for microorganism detection of the present disclosure, the presence or absence of a specific microorganism can be quickly determined only through visual inspection.

Examples of the specific microorganism mentioned above may comprise a bacterium, a fungi, etc., but they are not limited thereto. The bacterium may comprise, but is not limited to a lactic acid bacterium, *Escherichia coli,* a bacterium belonging to the genus *Salmonella, Staphylococcus aureus,* a bacterium belonging to the genus *Listeria* or a bacterium belonging to the genus *Shigella,* etc. Moreover, examples of the lactic acid bacterium mentioned above may comprise *Lactobacillus reuteri, Lactobacillus pentosus, Lactobacillus gasseri, Lactobacillus salivarius, Lactobacillus paracasei, Lactobacillus plantarum,* any combination thereof, but they are not limited thereto.

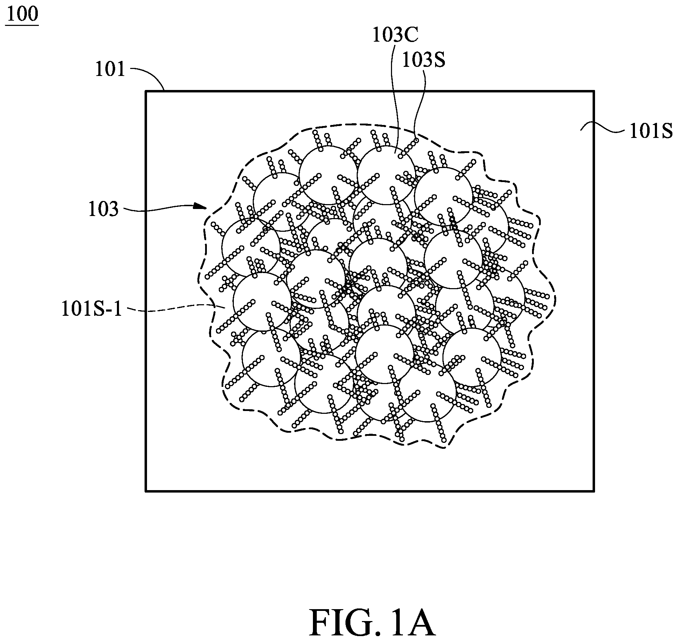

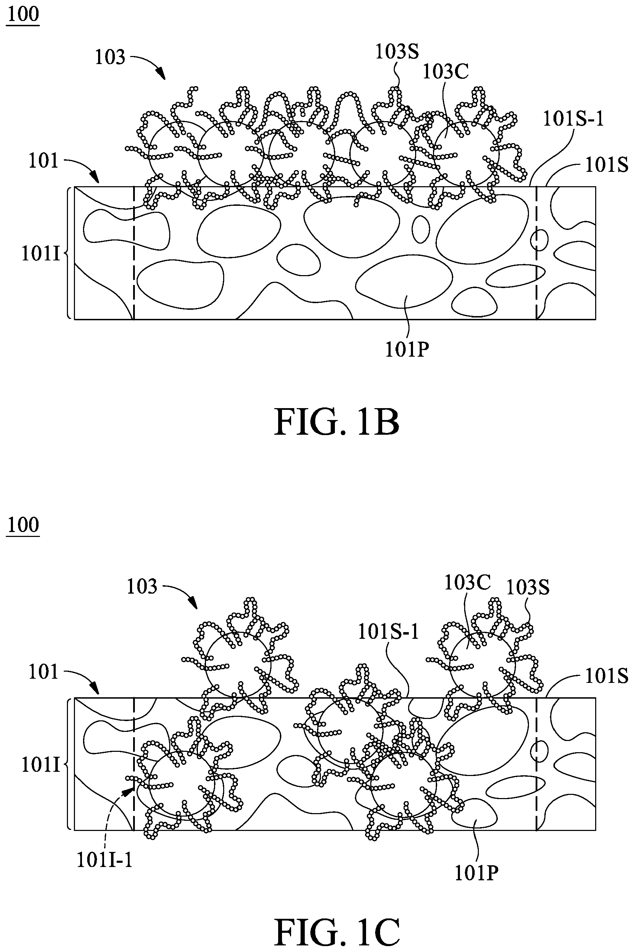

In order make the description more clear, in the following, the composition and structure of the test strip for microorganism detection of the present disclosure is illustrated by FIG. 1 to FIG. 5. However, it should be noted that the test strip for microorganism detection of present disclosure is not limited to the figures mentioned above.

FIGS. 1A-1C are schematic diagrams of the test strip for microorganism detection 100 of the present disclosure. FIG. 1A shows a top view of the test strip for microorganism detection 100 of the present disclosure. FIG. 1B shows a sectional view of the structure of the test strip for microorganism detection 100 in one embodiment of the present disclosure while FIG. 1C shows a sectional view of the structure of the test strip for microorganism detection 100 in another embodiment of the present disclosure.

Refer to FIGS. 1A-1C. The test strip for microorganism detection 100 of the present disclosure may comprise, but is not limited to a porous substrate 101 and a microorganism detection composition 103. A surface 101S of the foregoing porous substrate 101 has a first color. The microorganism detection composition 103 is at least adhered to a part 101S-1 of a surface 101S of the foregoing porous substrate 101 (referring to FIG. 1B) or at least adhered to a part 101S-1 of a surface 101S of the foregoing porous substrate 101 and a part 101I-1 of an interior 101I of the foregoing porous substrate 101 (referring to FIG. 1C).

Material suited for being used as the material for the porous substrate 101 of the present disclosure may be any porous and has no particular limitation. In one embodiment, the material for the porous substrate 101 may comprise a natural fiber material, an artificial fiber material, etc., but it is not limited thereto. Examples of the natural fiber material mentioned above may comprise, but are not limited to, paper, cotton, hemp, silk, a combination thereof and the like. Examples of the artificial fiber material mentioned above may comprise nitrocellulose, cellulose acetate, polyester, a combination thereof and the like, but they are not limited thereto. In one specific embodiment, the material for the porous substrate 101 may be nitrocellulose.

Furthermore, a pore size of the material for the porous substrate 101 may be about 0.01 μm-5.00 μm, such as about 0.01 μm-0.05 μm, about 0.05 μm-1.00 μm, about 1.00 μm-5.00 μm, etc., but it is not limited thereto.

In the test strip for microorganism detection 100 of the present disclosure, microorganism detection composition 103 mentioned above may comprise a plurality of colored particles 103C and a plurality of specific peptides 103S, but it is not limited thereto. The colored particle 103C mentioned above has a second color that is different than the first color. The foregoing plurality of specific peptides 103S are immobilized on the surfaces of the plurality of colored particles 103C. The foregoing plurality of specific peptides 103S may connect the plurality of colored particles 103C to each other to make the plurality of colored particles 103C aggregate to enable the microorganism detection composition 103 mentioned above cover the part 101S-1 of the surface 101S of the porous substrate 101 to which the microorganism detection composition 103 is adhered to and present the second color on the foregoing part 101S-1 of the surface 101S of the porous substrate 101.

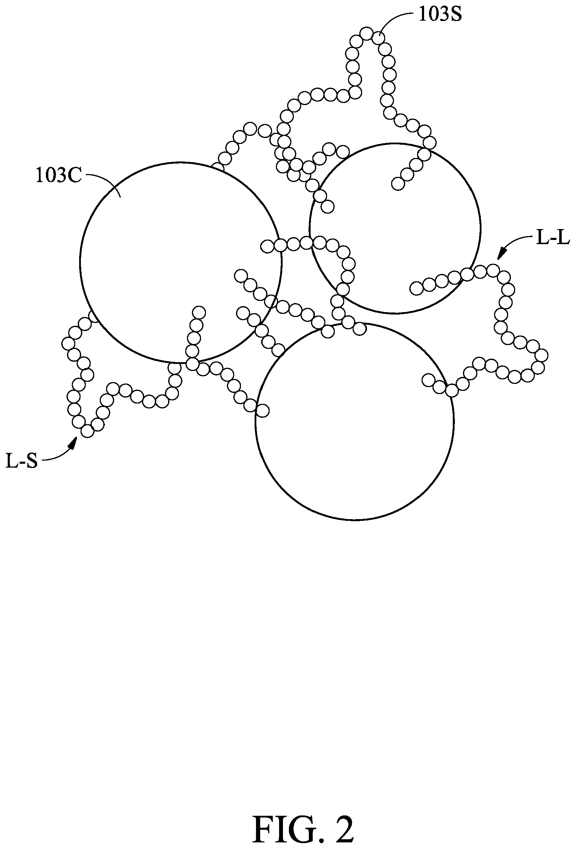

FIG. 2 shows a schematic diagram of the plurality of colored particles 103C being connected to each other by the plurality of specific peptides 103S in the microorganism detection composition 103. According to FIG. 2, it can be known that when the two ends of the specific peptide 103S are respectively immobilized on the surfaces of two different particles of the plurality of colored particles 103C, an inter-link L-L is formed between the two different particles, and when the two ends of the specific peptide 103S are respectively immobilized on the surface of the same particle of the plurality of colored particles 103C, the same particle forms a self-link L-S.

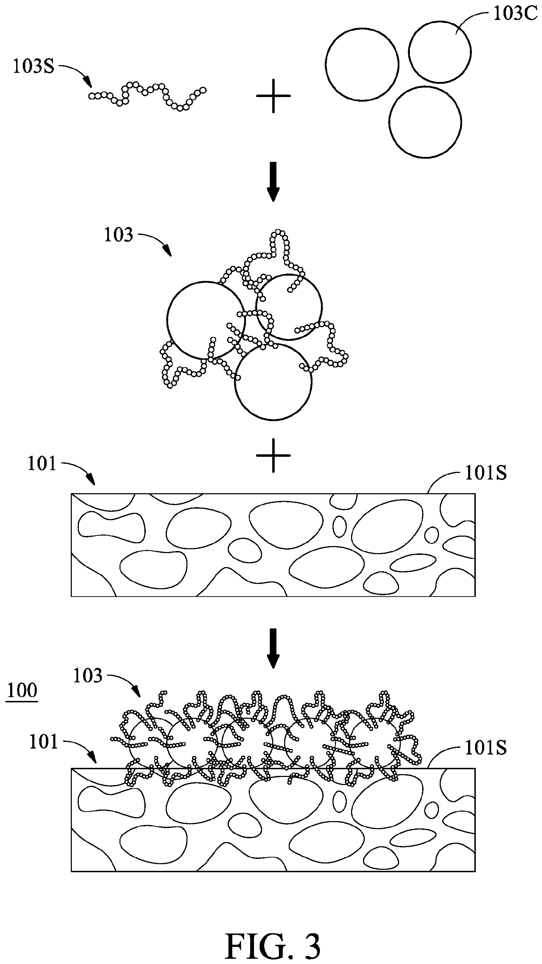

FIG. 3 is a schematic diagram showing one embodiment of a mode for forming the test strip for microorganism detection 100 of the present disclosure shown in FIG. 1B. First, a plurality of colored particles 103C are contacted with a plurality of specific peptides 103S to form a microorganism detection composition 103. Next, the microorganism detection composition 103 is contacted with the porous substrate 101 to make the microorganism detection composition 103 adhere to the surface 101S of the porous substrate 101. The manner of bringing the microorganism detection composition 103 into contact with the porous substrate 101 is not particularly limited, as long as the microorganism detection composition 103 can adhere to the surface of the porous substrate 101. For example, the microorganism detection composition 103 can be caused to make contact with the porous substrate 101 by way of inkjet, spotting, soaking, etc. to make the microorganism detection composition 103 adhere to the surface of the porous substrate 101, but it is not limited thereto.

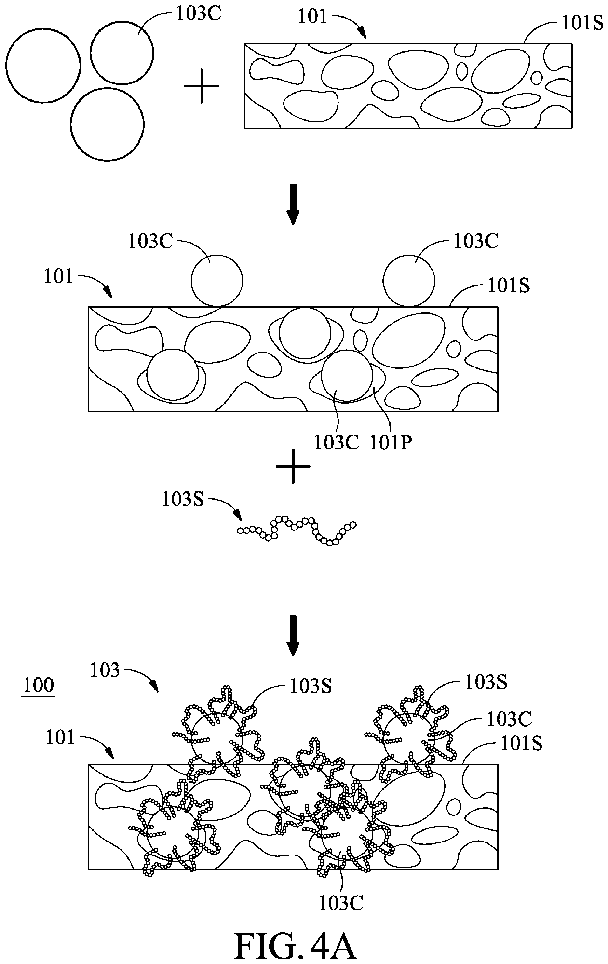

FIGs. 4A-4C are schematic diagrams showing different embodiments of a mode for forming the test strip for microorganism detection 100 of the present disclosure shown in FIG. 1C.

Refer to FIG. 4A and 1C. In the embodiment of FIG. 4A, first, the plurality of colored particles 103C are contacted with the porous substrate 101 to make the plurality of colored particles 103C attach to the surface 101S of the porous substrate 101 and enter the pores 101P of the porous substrate 101. The manner of bringing the plurality of colored particles 103C into contact with the porous substrate 101 is not particularly limited, as long as it enables the plurality of colored particles 103C to attach to the surface 101S of the porous substrate 101 and/or enter the pores 101P of the porous substrate 101. For example, the plurality of colored particles 103C can be caused to make contact with the porous substrate 101 by way of inkjet, spotting or soaking, etc. to make the plurality of colored particles 103C adhere to the surface 101S of the porous substrate 101 and/or enter the pores 101P of the porous substrate 101, but it is not limited thereto. After that, the porous substrate 101 is contacted with the plurality of specific peptides 103S to make the plurality of specific peptides 103S also attach to the surface 101S of the porous substrate 101 and enter the pores 101P of the porous substrate 101 and to immobilize the plurality of specific peptides 103S on the surfaces of the plurality of colored particles 103C to make the microorganism detection composition 103 adhere to the surface 101S and the interior 101I of the porous substrate 101. Moreover, after the aforementioned step of bringing the plurality of colored particles 103C into contact with the porous substrate 101, the manner of bringing the plurality of specific peptides 103S into contact with the porous substrate 101 is also not particularly limited, as long as it enables the plurality of specific peptides 103S to attach to the surface 101S of the porous substrate 101 and/or enter the pores 101P of the porous substrate 101, and connect to the plurality of colored particles 103C. For example, the plurality of specific peptides 103S can be caused to make contact with the porous substrate 101 by way of inkjet, spotting or soaking, etc. to make the plurality of specific peptides 103S adhere to the surface 101S of the porous substrate 101 and/or enter the pores 101P of the porous substrate 101, but it is not limited thereto.

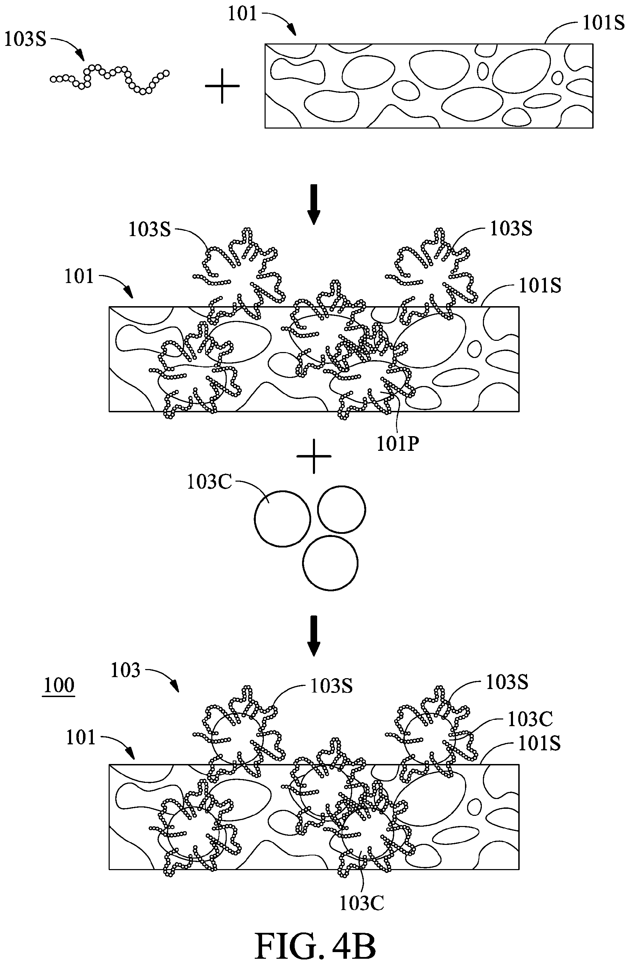

Furthermore, refer to FIG. 4B and 1C. In the embodiment of FIG. 4A, first, the plurality of specific peptides 103S are contacted with the porous substrate 101 to make the plurality of specific peptides 103S attach to the surface 101S of the porous substrate 101 and enter the pores 101P of the porous substrate 101. The manner of bringing the plurality of specific peptides 103S into contact with the porous substrate 101 is not particularly limited, as long as it enables the plurality of specific peptides 103S to attach to the surface 101S of the porous substrate 101 and/or enter the pores 101P of the porous substrate 101. For example, the plurality of specific peptides 103S can be caused to make contact with the porous substrate 101 by way of inkjet, spotting or soaking, etc. to make the plurality of specific peptides 103S adhere to the surface 101S of the porous substrate 101 and/or enter the pores 101P of the porous substrate 101, but it is not limited thereto. After that, the porous substrate 101 is contacted with the plurality of colored particles 103C to make the plurality of colored particles 103C also attach to the surface 101S of the porous substrate 101 and enter the pores 101P of the porous substrate 101 and to immobilize the plurality of specific peptides 103S on the surfaces of the plurality of colored particles 103C to make the microorganism detection composition 103 adhere to the surface 101S and the interior 101I of the porous substrate 101. Moreover, after the aforementioned step of causing the plurality of specific peptides 103S to come into contact with the porous substrate 101, the manner of bringing the plurality of colored particles 103C into contact with the porous substrate 101 is also not particularly limited, as long as it enables the plurality of colored particles 103C to attach to the surface 101S of the porous substrate 101 and/or enter the pores 101P of the porous substrate 101, and connect to the plurality of specific peptides 103S. For example, the plurality of colored particles 103C can be caused to make contact with the porous substrate 101 by way of inkjet, spotting or soaking, etc. to make the plurality of colored particles 103C adhere to the surface 101S of the porous substrate 101 and/or enter the pores 101P of the porous substrate 101, but it is not limited thereto.

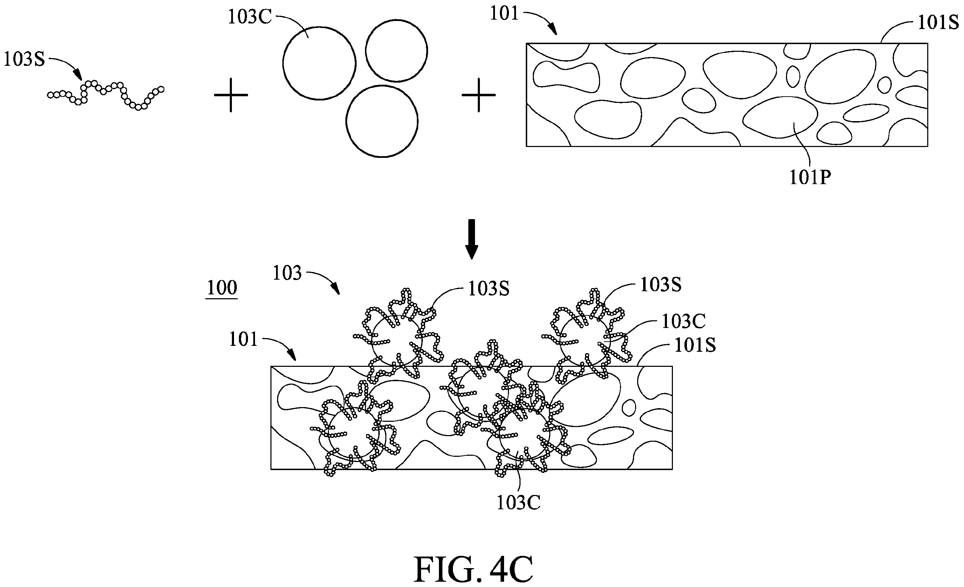

In addition, refer to FIG. 4C and 1C. In the embodiment of FIG. 4C, first, the plurality of colored particles 103C, the plurality of specific peptides 103S and the porous substrate 101 are contacted with each other at the same time to make the plurality of colored particles 103C and the plurality of specific peptides 103S attach to the surface 101S of the porous substrate 101 and enter the pores 101P of the porous substrate 101 and to immobilize the plurality of specific peptides 103S on the surfaces of the plurality of colored particles 103C to make the microorganism detection composition 103 adhere to the surface 101S and the interior 101I of the porous substrate 101. The manner of making the plurality of colored particles 103C, the plurality of specific peptides 103S and the porous substrate 101 comes into physical contact with each other at the same time is not particularly limited, as long as it enables the plurality of colored particles 103C and the plurality of specific peptides 103S to attach to the surface 101S of the porous substrate 101 and/or enter the pores 101P of the porous substrate 101. For example, the plurality of colored particles 103C, the plurality of specific peptides 103S and the porous substrate 101 can be soaked in a liquid at the same time to make the plurality of colored particles 103C and the plurality of specific peptides 103S adhere to the surface 101S of the porous substrate 101 and/or enter the pores 101P of the porous substrate 101 and connect to each other, but it is not limited thereto.

The shape of the colored particle 103C in the microorganism detection composition 103 has not particular limitation, for example, the shape of the colored particle may comprise a spherical shape, a square shape, a tapered shape, a rod shape, a polygonal shape, or the like, but is not limited thereto. In one embodiment, the shape of the colored particle may be a spherical shape.

Furthermore, the material of the colored particles 103C in the microorganism detection composition 103 is also not particularly limited, as long as it is not easily dissolved in an aqueous solution. Examples of the material of the colored particle 103C may comprise, but are not limited to a magnetic substance, glass, a polymer material, plastics, nano particles, combinations thereof and the like. In one embodiment, the material of the colored particles may be a magnetic substance so that it can be separated from the test sample and/or recovered by simple magnetic adsorption in the subsequent step.

In addition, the particle size of the colored particle 103C in the microorganism detection composition 103 mentioned may be about 0.01 $\mu$m-1 $\mu$m, such as about 0.01 $\mu$m-0.1 $\mu$m, 0.1 $\mu$m-0.5 $\mu$m, 0.5 $\mu$m-1 $\mu$m, etc., but it is not limited thereto.

Furthermore, in one embodiment, the colored particles 103C in the microorganism detection composition 103 may be subjected to a chemical treatment so that the specific peptide 103S in the microorganism detection composition 103 can be easily fixed thereon. The foregoing chemical treatment may enable the surface of the colored particle 103C mentioned above be modified with at least one functional group, but is not limited thereto. The type of at least one functional group modified on the surface of the colored particle 103C mentioned above is not particularly limited, and may depend on needs. For example, the type of at least one functional group modified on the surface of the above colored particle 103C may depend on the modification desired to be performed on the specific peptide 103S to be immobilized on the colored particle 103C. Examples of the at least one functional group mentioned above may comprise -COOH, -NH$_2$, -SH, and the like, but are not limited thereto. In one embodiment, the surface of the foregoing colored particle is modified with -COOH.

In addition, in the aforementioned microorganism detection composition 103, the specific peptide 103S is designed for the specific microorganism to be detected. The foregoing specific microorganism has the ability to cleave this specific peptide. For example, the specific microorganism to be detected can produce a specific enzyme which can be a secreted or membrane protein form, and the specific peptide 103S mentioned above is designed for this specific enzyme. Specifically, by designing a specific cleavage site for the specific enzyme in the above-mentioned specific peptide 103S, the above-specific peptide mentioned can be specifically recognize and cut by the specific enzyme produced by the specific microorganism.

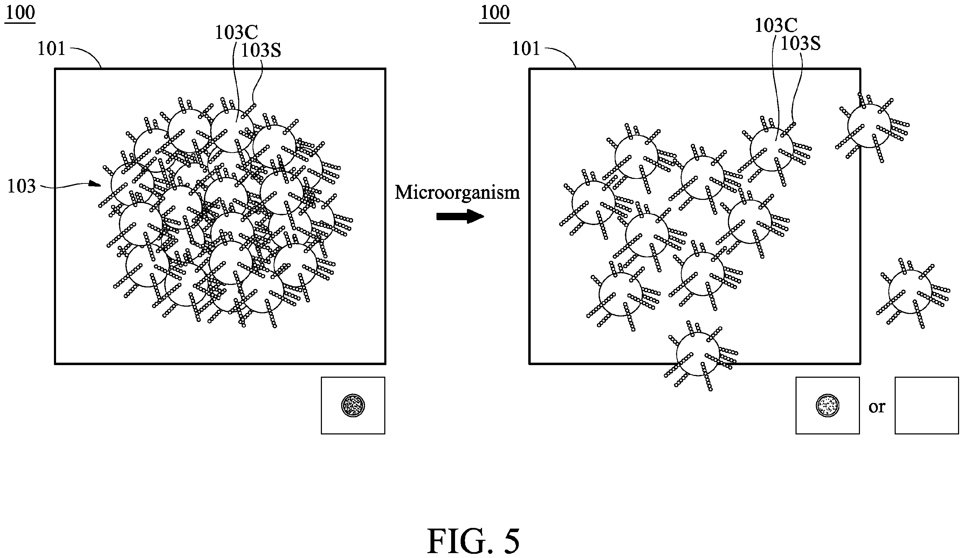

With regard to the usage scenario for the test strip for the microorganism detection 100 of present disclosure, please refer to FIG. 5. Before a detection for a testing sample is performed (referring to the left side of FIG. 5), since the plurality of colored particles 103C in the microorganism detection composition 103 are connected to each other by the plurality of peptides 103S and aggregate, the microorganism detection composition 103 covering at least one part 101S-1 of the surface 101S of the porous substrate 101 makes the part 101S-1 of the surface 101S of the test strip for microorganism detection 100 present the second color mentioned above (i.e. the color of the plurality of colored particles 103C) (referring to the small box of the left side of FIG. 5), and when the specific microorganism exists in the test sample, the specific microorganism will cleave the plurality of specific peptides 103S in the microorganism detection composition 103 which make the plurality of colored particles 103C be connected to each other, so that the aggregation of the plurality of colored particles 103C is reduced or even the plurality of colored particles 103C do not aggregate any more, and this cause that the above second color (i.e. the color of the plurality of colored particles 103C) presented on the part 101S-1 of the surface 101S of the porous substrate 101 becomes light and/or the part 101S-1 of the surface 101S of the porous substrate 101 is exposed to present the first color of the porous substrate 101 (referring to the small box of the right side of FIG. 5).

In the aforementioned microorganism detection composition 103, the length of the specific peptide 103S may be about 10-200 amino acids, such as about 10-150 amino acids, 10-100 amino acids, about 20-50 amino acids, about 15-25 amino acids, about 25-35 amino acids, about 30-40 amino acids, but it is not limited thereto.

In one embodiment, the N-terminal end and the C-terminal end of the specific peptide 103S may be respectively modified with a first functional group and a second functional group to make the specific peptide be easily immobilized on the surface of the colored particle 103C. The first functional group and the second functional group mentioned above may be homo-functional functional groups and the two may be the same or different while the first functional group and the second functional group mentioned above also may be hetero-functional functional groups. The types of the first functional group and the second functional group are not particularly limited and may depend on needs. For example, the type of the first functional group and the second functional group may depend on the modification desired to be performed on the colored particle 103C. For instance, the first functional group and the second functional group may independently comprise -NH$_2$, -COOH, biotin, -SH, etc., but they are not limited thereto. In a specific embodiment, the first functional group and the second functional group may both be -NH$_2$.

In one embodiment, the test strip for microorganism detection 100 of the present disclosure is used for detecting the presence of a lactic acid bacterium. The lactic acid bacterium may comprise, but is not limited to *Lactobacillus reuteri, Lactobacillus pentosus, Lactobacillus gasseri, Lactobacillus salivarius, Lactobacillus paracasei, Lactobacillus plantarum,* any combination thereof, etc.

In the foregoing embodiment in which the test strip for microorganism detection 100 of the present disclosure is used for detecting the presence of a lactic acid bacterium, the sequence of the specific peptide 103S of the microorganism detection composition 103 may comprise at least a part of the sequence of casein.

Moreover, in the foregoing embodiment in which the test strip for microorganism detection 100 of the present disclosure is used for detecting the presence of a lactic acid bacterium, the sequence of the specific peptide 103S of the microorganism detection composition 103 may comprise a sequence having at least about 75% sequence identity to the amino acid sequence of SEQ ID NO. 1, SEQ ID NO. 2, SEQ ID NO. 3, SEQ ID NO. 4 or SEQ ID NO. 5. For example, the sequence of the specific peptide 103S of the microorganism detection composition 103 may comprise the amino acid sequence of SEQ ID NO. 1, SEQ ID NO. 2, SEQ ID NO. 3, SEQ ID NO. 4 or SEQ ID NO. 5, but it is not limited thereto.

"At least about 75% sequence similarity" as described herein means that there is at least about 75% sequence similarity between two sequences, such as 80%, 81%, 82%, 85%, 90%, 95%, 99%, 99.5%, 100% sequence similarity, but it is not limited thereto.

In the foregoing embodiment in which the test strip for microorganism detection 100 of the present disclosure is used for detecting the presence of a lactic acid bacterium, in one specific embodiment, the sequence of the specific peptide 103S of the microorganism detection composition 103 may comprise the amino acid sequence of SEQ ID NO. 1. Moreover, in this specific embodiment, the sequence of the specific peptide 103S mentioned above may be the amino acid sequence of SEQ ID NO. 1 or SEQ ID NO. 6.

Furthermore, in the foregoing embodiment in which the test strip for microorganism detection 100 of the present disclosure is used for detecting the presence of a lactic acid bacterium, in one specific embodiment, the sequence of the specific peptide 103S of the microorganism detection composition 103 may comprise the amino acid sequence of SEQ ID NO. 2. Moreover, in this specific embodiment, the sequence of the specific peptide 103S mentioned above may be the amino acid sequence of SEQ ID NO. 2 or SEQ ID NO. 7.

Furthermore, in the foregoing embodiment in which the test strip for microorganism detection 100 of the present disclosure is used for detecting the presence of a lactic acid bacterium, in one specific embodiment, the N-terminal end and the C-terminal end of the specific peptide 103S of the microorganism detection composition 103 are both modified with -NH$_2$ group. Moreover, the surface of the colored particle 103C of the microorganism detection composition 103 in the test strip for microorganism detection 100 is modified with -COOH group, and the colored particle 103C is a magnetic bead. In addition, the material of the porous substrate 101 of the test strip for microorganism detection 100 is nitrocellulose.

The present disclosure also provides a microorganism detection method. The microorganism detection method of the present disclosure is used for detecting the presence of a specific microorganism.

Examples of the specific microorganism mentioned above may comprise a bacterium, a fungi, etc., but they are not limited thereto. The bacterium may comprise, but is not limited to a lactic acid bacterium, *Escherichia coli,* a bacterium belonging to the genus *Salmonella, Staphylococcus aureus,* a bacterium belonging to the genus *Listeria* or a bacterium belonging to the genus *Shigella,* etc. Moreover, examples of the lactic acid bacterium mentioned above may comprise *Lactobacillus reuteri, Lactobacillus pentosus, Lactobacillus gasseri, Lactobacillus salivarius, Lactobacillus paracasei, Lactobacillus plantarum,* any combination thereof, but they are not limited thereto.

The microorganism detection method of the present disclosure may comprise the following steps, but it is not limited thereto.

A test sample is contacted with any test strip for microorganism detection 100 of the preset disclosure.

The test sample is not particularly limited, as long as it is an object that may contain microorganisms. In one embodiment, the test sample may comprise foods, such as fresh food, canned food, packaged beverage, etc., but is not limited thereto.

The terms "contact", "contacting", "is contacted to" or "in contact with" in the present disclosure do not mean that the test sample must be in direct contact with the test strip for microorganism detection 100 of the present disclosure, as long as a scenario in which the test strip for microorganism detection 100 of the present disclosure can react with the test sample and/or an enzyme released by a bacterium in the test sample can be achieved. In one embodiment, the test sample may be a meat stored at a low temperature, and the test strip for microorganism detection 100 of the present disclosure and the meat stored at a low temperature are packaged together in a sealed package, and that enables the test strip for microorganism detection 100 to react with the test sample and/or an enzyme released by a bacterium in the test sample without direct contact of the meat with the test strip for microorganism detection 100 of the present disclosure.

There is no particular limitation in the time needed for the test sample to be in contact with the test strip for microorganism detection 100 of the present disclosure, as long as the test sample can be sufficiently reacted with the test strip for microorganism detection 100 of the present disclosure. At room temperature (about 20-28°C), the time during which the test sample should be in contact with the test strip for microorganism detection 100 of the present disclosure may be about 7 days or less, such as about 6 hours to 7 days, about 6-12 hours, 3-7 days, about 8 hours, about 12 hours, about 1 day, about 5 days, about 6 days, about 7 days, etc., but it is not limited thereto. In a low temperature refrigerated condition (about 2-8°C), the time during which the test sample should be in contact with the test strip for microorganism detection 100 of the present disclosure may be about 3 weeks or less, such as about 12 hours to 3 weeks, about 12 to 48 hours, about 1-3 weeks, about 18 hours, about 24 hours, about 1 week, about 2 weeks, about 3 weeks, etc., but it is not limited thereto.

After the test sample comes into contact with the test strip for microorganism detection, the color change of the test strip for microorganism detection is determined.

FIG. 5 is a schematic diagram showing the detection of a specific microorganism in a test sample by the microorganism detection method of the present disclosure.

As mentioned above, since the specific peptide 103S of the microorganism detection composition 103 in the test strip for microorganism detection 100 is designed for a specific microorganism desired to be detected, during detection of a specific microorganism by the microorganism detection method of the present disclosure, if the specific microorganism to be detected exists in the test sample, the specific microorganism will cleave the plurality of specific peptides 103S which connect the plurality of colored particles 103C to each other in the microorganism detection composition 103 of the test strip for microorganism detection 100, so that the aggregation of the plurality of colored particles 103C in the microorganism detection composition 103 is reduced or even the plurality of colored particles 103C do not aggregate any more in the microorganism detection composition 103, and this cause that the above second color (i.e. the color of the plurality of colored particles 103C) presented on the part 101S-1 of the surface 101S of the porous substrate 101 becomes light and/or the part 101S-1 of the surface 101S of the porous substrate 101 is exposed to present the first color of the porous substrate 101 (referring to the small box of the right side of FIG. 5). On the contrary, if the microorganism to be detected does not exist in the test sample, the plurality of colored particles 103C in the microorganism detection composition 103 of the test strip for microorganism detection 100 of the present disclosure will still maintain aggregation so that the second color (i.e. the color of the plurality of colored particles 103C) is still maintained on the part 101S-1 of the surface 101S of the porous substrate 101 of the test strip for microorganism detection 100 of the present disclosure.

Since the microorganism detection method of the present disclosure only needs to use the test strip for microorganism detection 100 of the present disclosure, and can quickly determine the presence or absence of a specific microorganism by only visual inspection, it has the advantages of convenience and ease of operation.

Moreover, in the microorganism detection method of the present disclosure, the foregoing test sample may be in a liquid form, but is not limited thereto.

Furthermore, there is no particular limitation in the temperature at which the test sample should be in contact with the test strip for microorganism detection 100 of the present disclosure, as long as the specific microorganism being detected can react with the specific peptide 103S in the test strip for microorganism detection 100 of the present disclosure. In one embodiment, the test sample comes into contact with the test strip for microorganism detection 100 at a temperature of about 2-56°C, such as about 4-10°C, about 10-20°C, about 20-40°C, about 40-56°C, etc., but it is not limited thereto. In one specific embodiment, the contact of the test sample with the test strip for microorganism detection 100 is performed at about 4°C, 25°C, 37°C or 56°C.

In one embodiment, the microorganism detection method is used for detecting the presence of a lactic acid bacterium. The lactic acid bacterium may comprise, but is not limited to *Lactobacillus reuteri, Lactobacillus pentosus, Lactobacillus gasseri, Lactobacillus salivarius, Lactobacillus paracasei, Lactobacillus plantarum,* any combination thereof, etc.

In the foregoing embodiment in which the microorganism detection method is used for detecting the presence of a lactic acid bacterium, it is needed to use a test strip for microorganism detection 100 of the present disclosure suited for the detection of lactic acid bacteria.

In the foregoing embodiment in which the microorganism detection method is used for detecting the presence of a lactic acid bacterium, with regard to the test strip for microorganism detection 100 suited for the detection of lactic acid bacteria, the sequence of the specific peptide 103S of the microorganism detection composition 103 may comprise at least a part of the sequence of casein.

Moreover, in the foregoing embodiment in which the microorganism detection method is used for detecting the presence of a lactic acid bacterium, with regard to the test strip for microorganism detection 100 suited for the detection of lactic acid bacteria, the sequence of the specific peptide 103S of the microorganism detection composition 103 may comprise a sequence having at least about 75% sequence identity to the amino acid sequence of SEQ ID NO. 1, SEQ ID NO. 2, SEQ ID NO. 3, SEQ ID NO. 4 or SEQ ID NO. 5. For example, the sequence of the specific peptide 103S of the microorganism detection composition 103 may comprise the amino acid sequence of SEQ ID NO. 1, SEQ ID NO. 2, SEQ ID NO. 3, SEQ ID NO. 4 or SEQ ID NO. 5, but it is not limited thereto.

"At least about 75% sequence similarity" as described herein means that there is at least about 75% sequence similarity between two sequences, such as 75%, 80%, 81%, 82%, 85%, 90%, 95%, 99%, 99.5%, 100% sequence similarity, but it is not limited thereto.

In the foregoing embodiment in which the microorganism detection method is used for detecting the presence of a lactic acid bacterium, in one specific embodiment, with regard to the test strip for microorganism detection 100 suited for the detection of lactic acid bacteria, the sequence of the specific peptide 103S of the microorganism detection composition 103 may comprise the amino acid sequence of SEQ ID NO. 1. Moreover, in this specific embodiment, the sequence of the specific peptide 103S mentioned above may be the amino acid sequence of SEQ ID NO. 1 or SEQ ID NO. 6.

Furthermore, in the foregoing embodiment in which the microorganism detection method is used for detecting the presence of a lactic acid bacterium, with regard to the test strip for microorganism detection 100 suited for the detection of lactic acid bacteria, in one specific embodiment, the sequence of the specific peptide 103S of the microorganism detection composition 103 may comprise the amino acid sequence of SEQ ID NO. 2. Moreover, in this specific embodiment, the sequence of the specific peptide 103S mentioned above may be the amino acid sequence of SEQ ID NO. 2 or SEQ ID NO. 7.

EXAMPLES

1. Example 1: Peptide designing

Lactic acid bacteria were set as the detection targets, the following 7 specific peptides of lactic acid bacteria were designed according to the sequence of casein, and Mission Biotech (Taiwan) and Bio Pioneer Tech CO., LTD (Taiwan) were entrusted to assist in the synthesis of these 7 specific peptides of lactic acid bacteria.

(1) For $\alpha$-casein

$\alpha$1-23-mer peptide (SEQ ID NO. 1); $\alpha$1-35-mer peptide (SEQ ID NO. 6).

(2) For $\beta$-casein

$\beta$2-23-mer peptide (SEQ ID NO. 2); $\beta$2-35-mer peptide (SEQ ID NO. 7); $\beta$3-23-mer peptide (SEQ ID NO. 3); $\beta$4-23-mer peptide (SEQ ID NO. 4); $\beta$5-23-mer peptide(SEQ ID NO. 5).

2. Example 2: Selection for substrate

Photocopy paper, filter paper (Whatman), nitrocellulose film (GE) and PVDF film (Millipore) were tested as porous substrates, and a magnetic bead suspension with a magnetic bead content of 7.5 mg/ml was prepared.

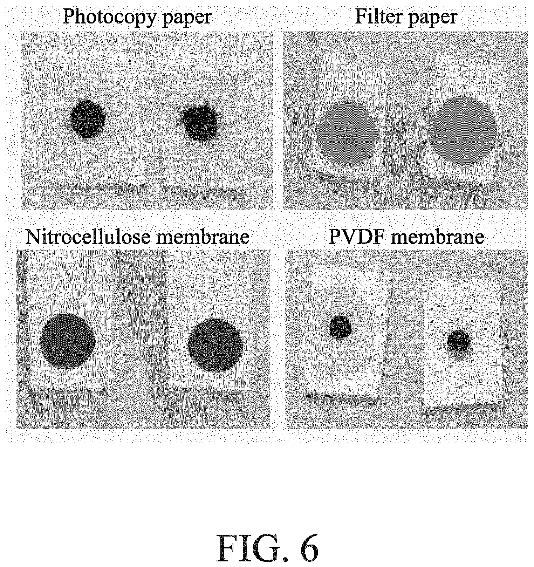

The magnetic bead suspension with a volume of 10 $\mu$l was dropped on the photocopy paper, the filter paper, the nitrocellulose film or the PVDF film, and the results are as shown in FIG. 6.

Based on FIG. 6, it can be known that after the magnetic bead suspension is dropped on the aforementioned substrates, the magnetic bead suspension can be diffused into a complete circular shape on the nitrocellulose film and the magnetic beads can be uniformly distributed on the nitrocellulose film. Moreover, although the magnetic bead suspension did not diffuse into a complete circle on the photocopy paper, the magnetic beads still could be uniformly distributed on the photocopy paper.

Overall, for all of the potential substrate materials that were tested, nitrocellulose film had the best diffusion performance. Since nitrocellulose film has properties such as porosity, negative charge, easy adsorption of protein and peptides, and is often used as a substrate material in many medical test strips, the nitrocellulose film was selected as the ideal material for use in the subsequent experiments.

Example 3: Preparation of test strip for microorganism detection

3.1 Method A

3.1.1 Preparation of test strip for microorganism detection containing magnetic beads on which peptides without modification are immobilized

Magnetic beads activated by 1-ethyl-3-(3-dimethyl aminopropyl) carbodiimide hydrochloride/N-Hydroxysuccinimide (EDC/NHS) were mixed with the peptides ($\alpha$1-23-mer peptide or $\beta$2-23-mer peptide), and then the mixture was dropped on a nitrocellulose film.

3.1.2 Preparation of test strip for microorganism detection containing magnetic beads on which peptides with modification are immobilized

Magnetic beads activated by EDC/NHS were mixed with the peptides modified with $NH_2$ functional group ($\alpha$1-23-mer peptide, $\beta$2-23-mer peptide, $\alpha$1-35-mer peptide or $\beta$2-35-mer peptide of which the two ends were modified with $NH_2$ functional group) ($\alpha$1-23-mer peptide, $\beta$2-23-mer peptide, $\alpha$1-35-mer peptide and $\beta$2-35-mer peptide of which the two ends were modified with $NH_2$ functional group are also called $\alpha$1-23-mer-$NH_2$ peptide, $\beta$2-23-mer-$NH_2$ peptide, $\alpha$1-35-mer-$NH_2$ peptide and $\beta$2-35-mer-$NH_2$ peptide in the following, respectively), and then the mixture was dropped on a nitrocellulose film.

3.2 Method B

Preparation of test strip for microorganism detection containing magnetic beads on which peptides with modification are immobilized.

Magnetic beads activated by EDC/NHS, peptides modified with $NH_2$ functional group ($\alpha$1-23-mer-$NH_2$ peptide or $\beta$2-23-mer-$NH_2$ peptide) and a nitrocellulose film were brought into contact with each other at the same time and left to stand overnight. After that, a film full of magnetic beads was obtained.

4. Example 4

Scanning electron microscopy (SEM) analysis for test strip for microorganism detection

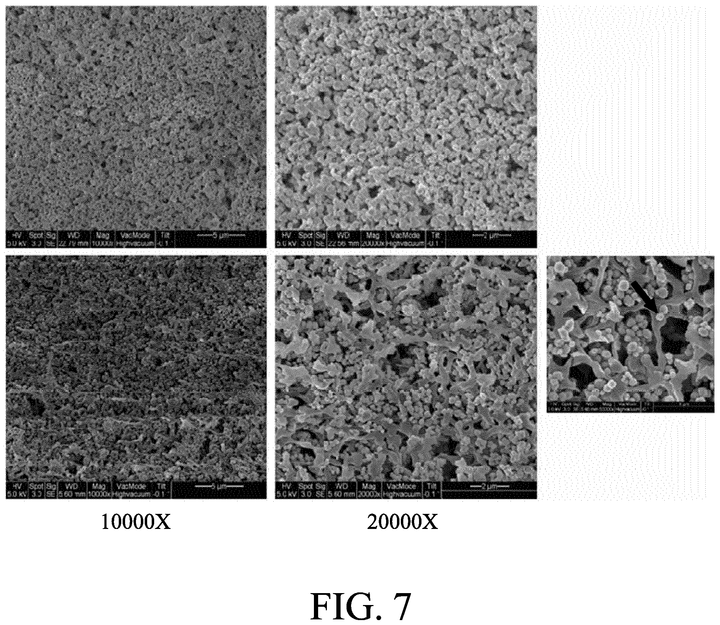

The test strips respectively obtained by Method A and Method B in Example 3 were analyzed by a scanning electron microscope, respectively, and the results are as shown in FIG. 7.

The upper block of FIG. 7 shows the result of scanning electron microscopy analysis of the test strip obtained by Method A (containing $\alpha$1-23-mer-$NH_2$ peptide) while the lower block of FIG. 7 shows the result of scanning electron microscopy analysis of the test strip obtained by Method B (containing $\alpha$1-23-mer-$NH_2$ peptide), wherein the magnification of the photograph on the left side is 10000X while the magnification of the photograph on the right side is 20000X.

According to the photograph on the upper block of FIG. 7, it can be known that the surface of the test strip obtained by Method A is covered with a layer of magnetic beads. The photographs on the lower block of FIG. 7 clearly show that magnetic beads can enter the pores of the nitrocellulose film in the test strip obtained by Method B.

5. Example 5: Enzymatic degradation test for test strip for microorganism detection containing magnetic beads on which peptides without modification are immobilized

Enzymatic degradation tests were performed on the test strips obtained by item 3.1.1 in Method A of Example 3 (containing magnetic beads on which $\alpha$1-23-mer peptide or $\beta$2-23-mer peptide without modification was immobilized) by proteinase K or trypsin with different dilution ratios. PBS buffer was used in the control group.

Image analysis was performed on the test strips before and after the test, and the image intensity (gray density) for the determining region was calculated (the value of all black was 255; the lighter the color was, the lower the value was; the value for all white was 0). Digestion level (DL) of peptide was calculated based on the image intensity. Calculation formula for digestion level of peptide is as shown in the following:

$$\text{Digestion level (DL)} = (\text{Image intensity of the region covered by the magnetic beads before the test - Image intensity of the region covered by the magnetic beads after the test})^2/\text{Area of the region covered by the magnetic beads before the test.}$$

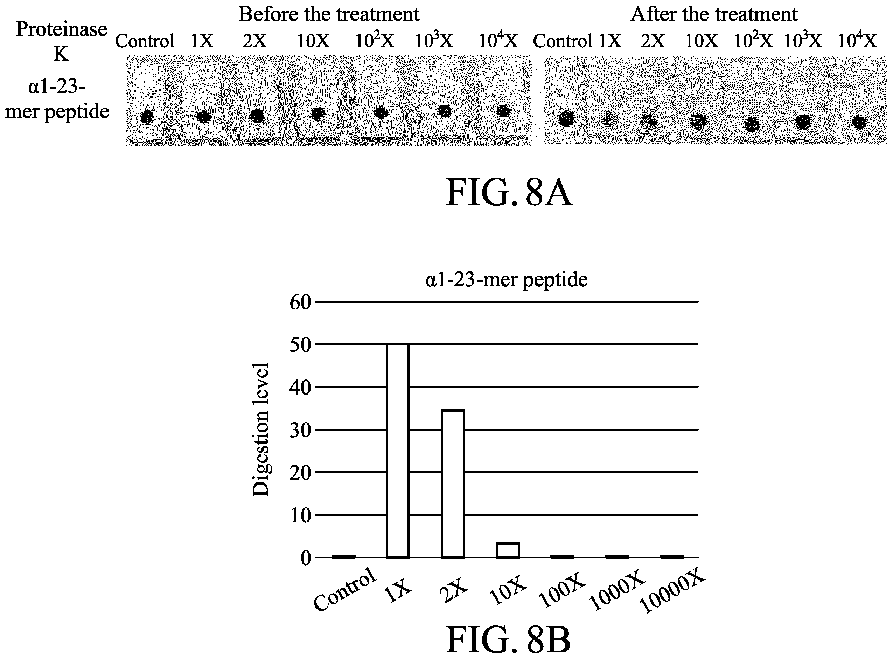

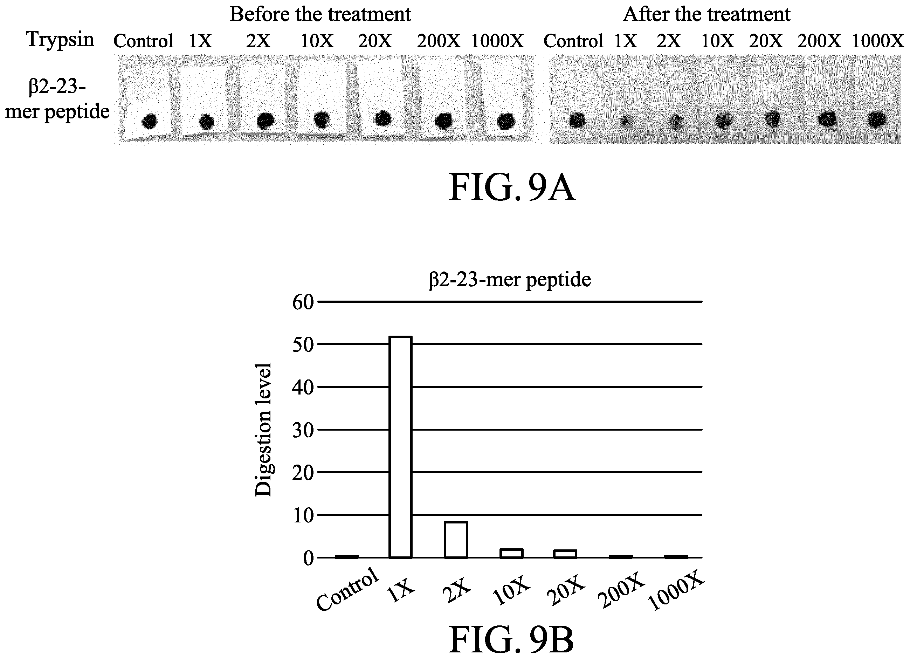

The results are as shown in FIGs. 8A and 8B and FIGs. 9A and 9B. FIGs. 8A and 8B show the results of the test strip containing magnetic beads on which $\alpha$1-23-mer peptide without modification is immobilized while FIGs. 9A and 9B show the results of the test strips containing magnetic beads on which $\beta$2-23-mer peptide without modification is immobilized.

Based on FIG. 8A, it can be known that as the dilution ratios of proteinase K increases, the stripping degree of the magnetic bead from the test strip containing magnetic beads on which $\alpha$1-23-mer peptide without modification is immobilized relatively decreases, and the result of analyzing the image intensity with ImageJ software is the same as the visual result. Moreover, based on FIG. 8B, it can be known that as the dilution ratios of proteinase K increases, the digestion level of peptide relatively decreases. The results mentioned above represent that the stripping of the magnetic beads on which $\alpha$1-23-mer peptide without modification is immobilized is caused by proteinase K.

Similarly, FIG. 9A shows that as the dilution ratios of trypsin increases, the stripping degree of the magnetic bead from the test strip containing magnetic beads on which $\beta$2-23-mer peptide without modification is immobilized relatively decreases, and FIG. 9B shows that as the dilution ratios of trypsin increases, the digestion level of peptide relatively decreases. The results mentioned above also represent that the stripping of the magnetic beads on which $\beta$2-23-mer peptide without modification is immobilized is caused by trypsin.

6. Example 6: Lactic acid bacteria test for test strip for microorganism detection containing magnetic beads on which peptides without modification are immobilized

The test strips obtained by item 3.1.1 in Method A of Example 3 (containing magnetic beads on which $\alpha$1-23-mer peptide or $\beta$2-23-mer peptide without modification was immobilized) were tested by six kinds of lactic acid bacteria (*Lactobacillus reuteri, Lactobacillus pentosus, Lactobacillus gasseri, Lactobacillus salivarius, Lactobacillus paracasei* or *Lactobacillus plantarum*). Each test was performed in duplication. PBS buffer was used in the control group.

The test strips were co-cultured with lactic acid bacteria in a 37°C incubator. After 3 days, the test strip was taken out and photographed, image analysis was performed by ImageJ software, and the image intensity (gray density) for the determining region was calculated (the value of all black was 255; the lighter the color was, the lower the value was; the value for all white was 0). Digestion level (DL) of peptide was calculated based on the image intensity. Three parameters, digestion level (DL) of peptide, area change (AC) of the region covered by the magnetic beads, and detachment level were calculated. Calculation formula for digestion level of peptide is as shown above while calculation formula for area change (AC) of the region covered by the magnetic beads and formula for detachment level are as shown in the following.

$$\text{Area change (AC) of the region covered by the magnetic beads} = [(\text{Area of the}$$

region covered by the magnetic beads before the test - Area of the region covered by the magnetic beads after the test)/Area of the region covered by the magnetic beads before the

$$\text{test}] \times 100;$$

$$\text{Detachment level} = \text{Digestion level (DL) of peptide} \times \text{Area change (AC) of the}$$

region covered by the magnetic beads.

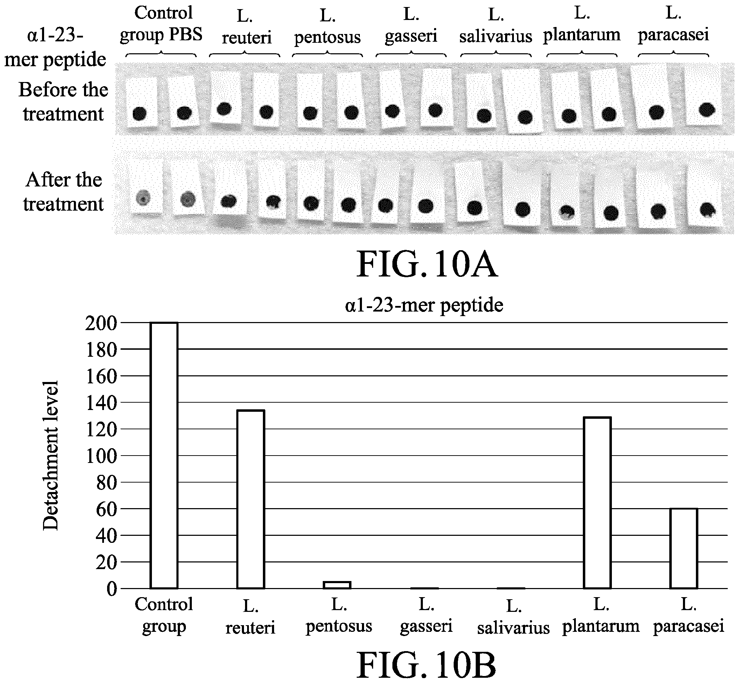

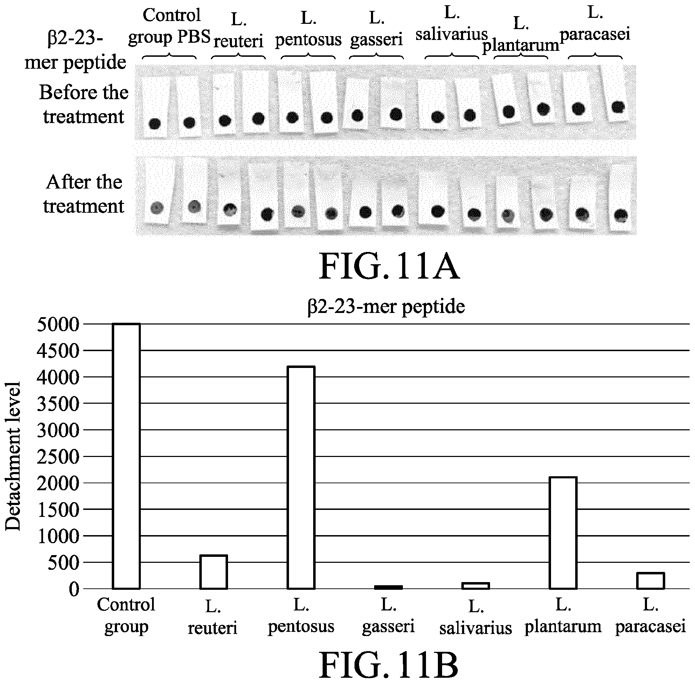

The results are as shown in FIGs. 10A and 10B and FIGs. 11A and 11B. FIGs. 10A and 10B show the test results for the test strip for microorganism detection containing magnetic beads on which $\alpha$1-23-mer peptide without modification was immobilized, and FIGs. 11A and 11B show the test results for the test strip for microorganism detection containing magnetic beads on which $\beta$2-23-mer peptide without modification was immobilized.

Based on FIGs. 10A and 10B, it can be known that *Lactobacillus reuteri, Lactobacillus plantarum* and *Lactobacillus paracasei* prefer $\alpha$1-23-mer peptide. Based on FIGs. 11A and 11B, it can be known that *Lactobacillus plantarum* and *Lactobacillus pentosus* prefer $\beta$2-23-mer peptide. *Lactobacillus plantarum, Lactobacillus paracasei, Lactobacillus pentosus* and *Lactobacillus reuteri* were selected as the material for the subsequent tests.

Example 7: Enzymatic degradation test for test strip for microorganism detection containing magnetic beads on which peptides with modification are immobilized

Enzymatic degradation tests were performed on the test strips obtained by item 3.1.2 in Method A and Method B of Example 3 (containing magnetic beads on which $\alpha$1-23-mer-NH$_2$ peptide, $\beta$2-23-mer-NH$_2$ peptide, $\alpha$1-35-mer-NH$_2$ peptide or $\beta$2-35-mer-NH$_2$ peptide was immobilized) by proteinase K or trypsin with different dilution ratios. Control group: PBS buffer.

Image analysis was performed on the test strips before and after the test, and two parameters, digestion level of peptide and area change (AC) of the region covered by the magnetic beads, were calculated. Calculation formulas for digestion level of peptide and area change (AC) of the region covered by the magnetic beads are shown as above.

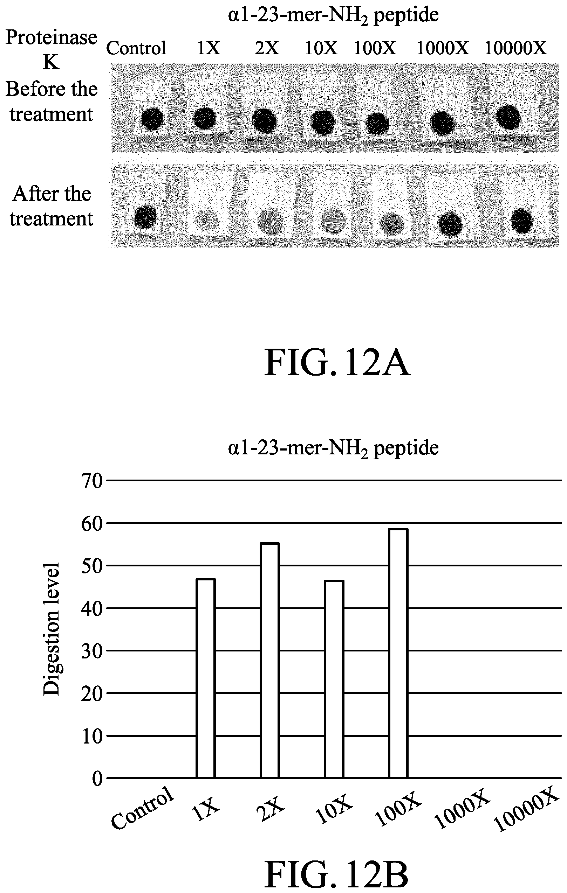

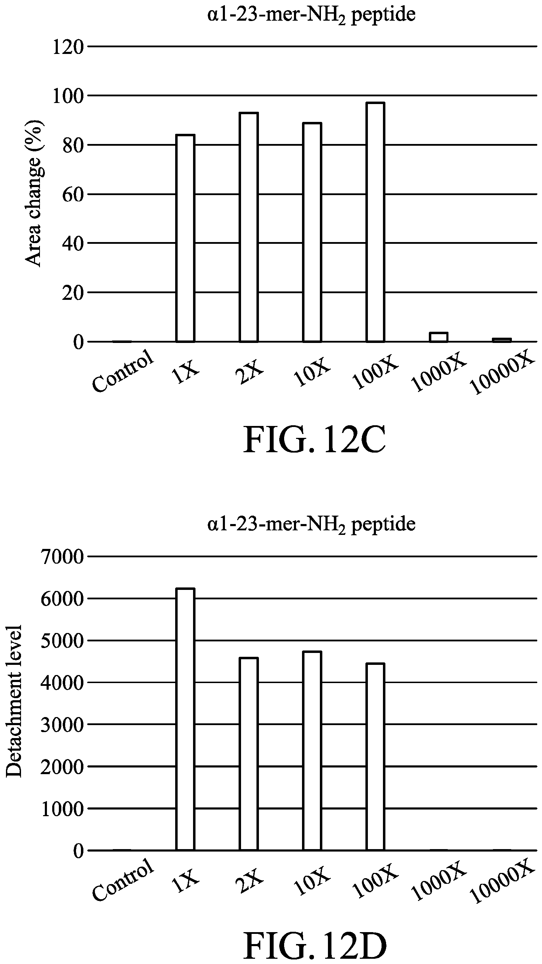

The results are as shown in FIGs. 12A to 12D, FIGs. 13A to 13D, FIG. 14, FIG. 15, FIGs. 16A and 16B and FIGs. 17A and 17B.

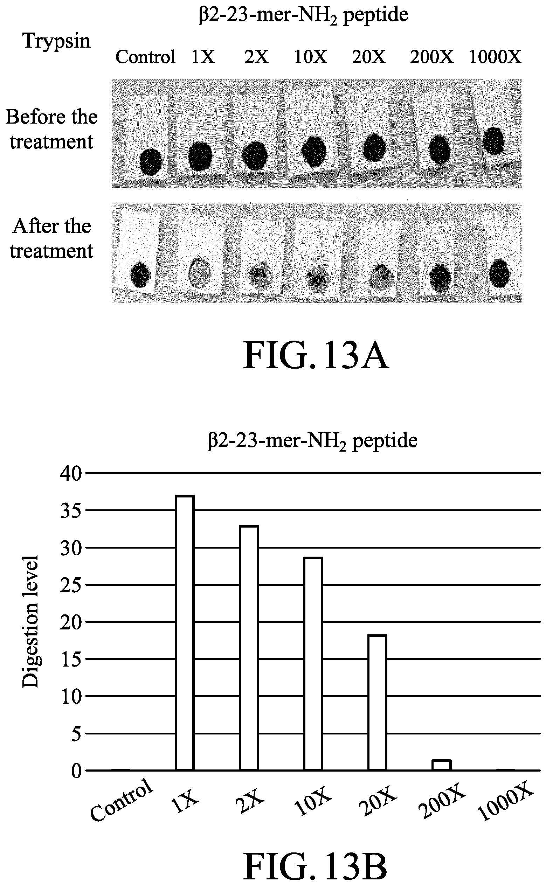

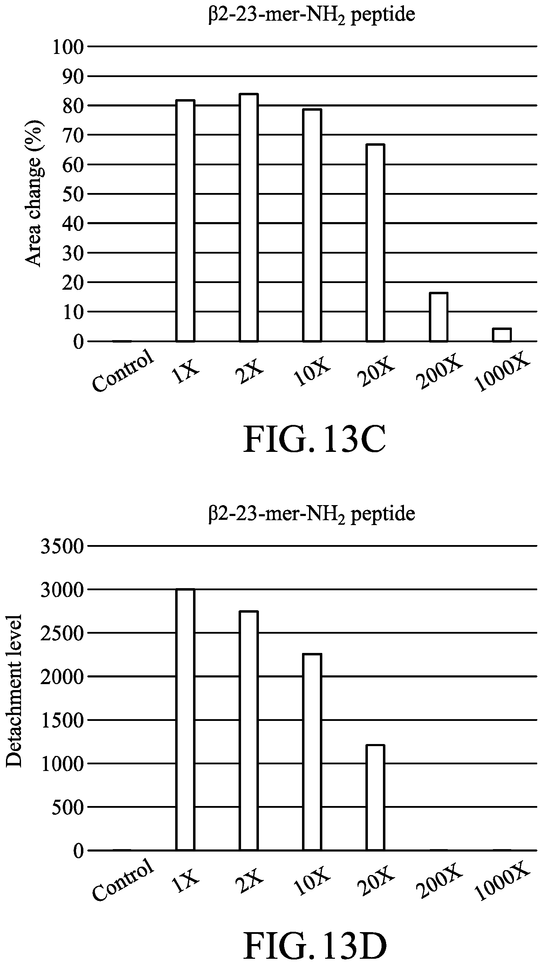

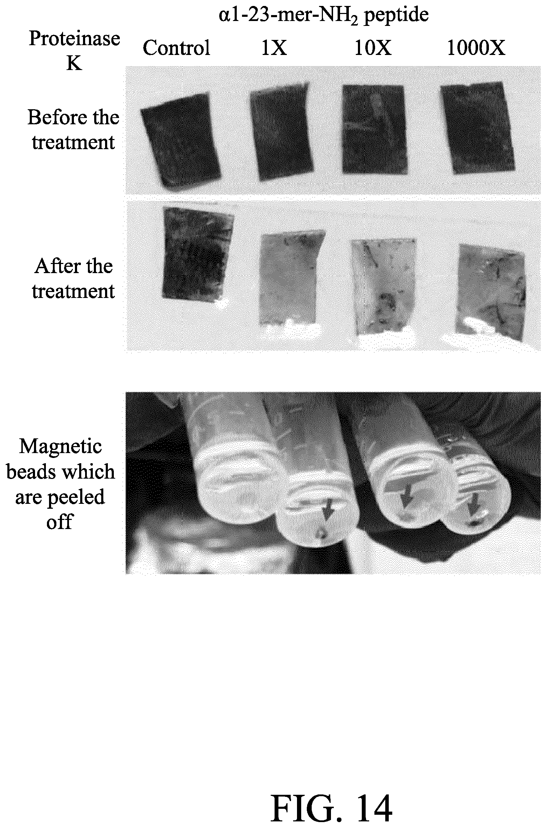

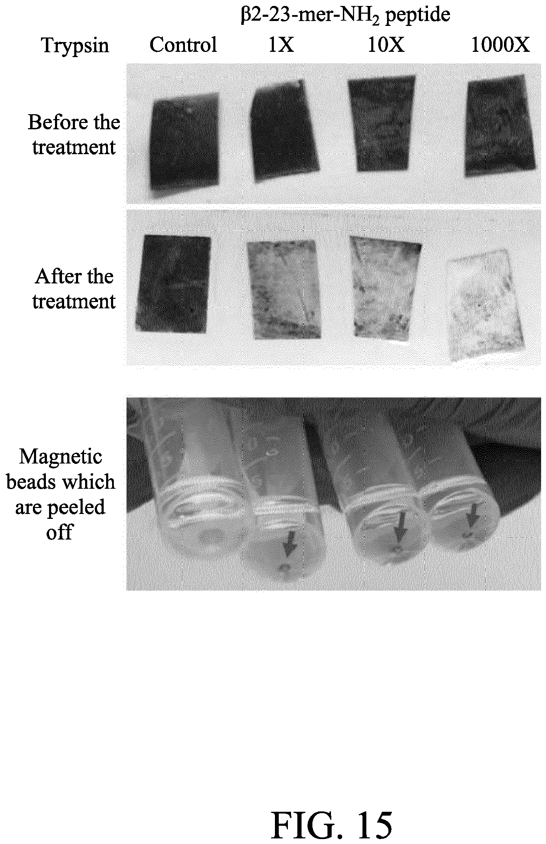

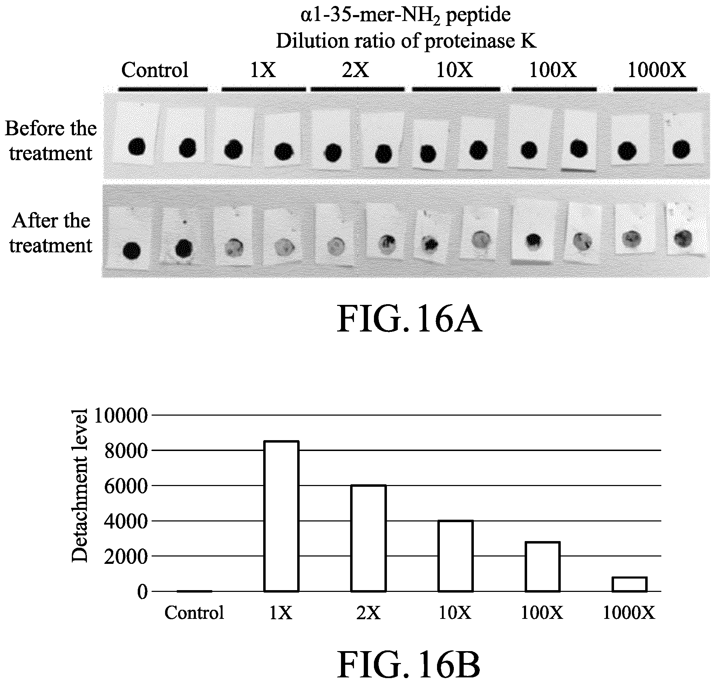

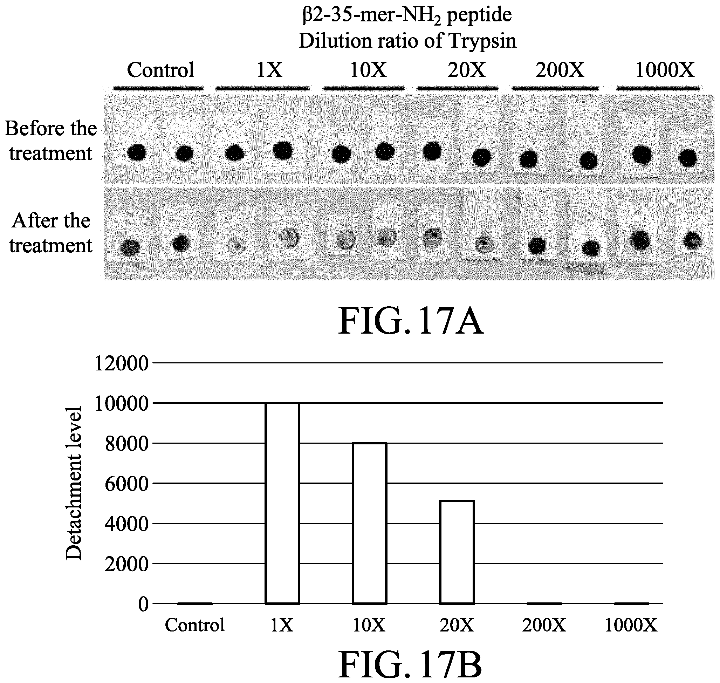

FIGs. 12A to 12D show the test results for the test strip containing magnetic beads on which $\alpha$1-23-mer-NH$_2$ peptide prepared by Method A was immobilized while FIGs. 13A to 13D show the test results for the test strip containing magnetic beads on which $\beta$2-23-mer-NH$_2$ peptide prepared by Method A was immobilized. FIG. 14 shows the test results for the test strip containing magnetic beads on which $\alpha$1-23-mer-NH$_2$ peptide prepared by Method B was immobilized while FIG. 15 shows the test results for the test strip containing magnetic beads on which $\beta$2-23-mer-NH$_2$ peptide prepared by Method B was immobilized. FIGs. 16A and 16B show the test results for the test strip containing magnetic beads on which $\alpha$1-35-mer-NH$_2$ peptide prepared by Method A was immobilized while FIGs. 17A and 17B show the test results for the test strip containing magnetic beads on which $\beta$2-35-mer-NH$_2$ peptide prepared by Method A was immobilized.

Based on FIGs. 12A to 12D, it can be known that as the dilution ratio of proteinase K increases, the stripping degree of the magnetic bead from the test strip containing magnetic beads on which $\alpha$1-23-mer-NH$_2$ peptide prepared by Method A was immobilized relatively decreases and the digestion level of peptide relatively decreases, and that represents that the stripping of the magnetic beads is caused by proteinase K. According to FIGs. 16A and 16B, it can be known that the test strip containing magnetic beads on which $\alpha$1-35-mer-NH$_2$ peptide prepared by Method A was immobilized has a results similar to that of the test strip containing magnetic beads on which $\alpha$1-23-mer-NH$_2$ peptide prepared by Method A was immobilized mentioned above.

In addition, based on FIGs. 13A to 13D, it can be known that as the dilution ratio of trypsin increases, the stripping degree of the magnetic bead from the test strip containing magnetic beads on which $\beta$2-23-mer-NH$_2$ peptide prepared by Method A was immobilized relatively decreases and the digestion level of peptide relatively decreases, and that represents that the stripping of the magnetic beads is caused by trypsin. According to FIGs. 17A and 17B, it can be known that the test strip containing magnetic beads on which $\beta$2-35-mer-NH$_2$ peptide prepared by Method A was immobilized has a results similar to that of the test strip containing magnetic beads on which $\beta$2-23-mer-NH$_2$ peptide

prepared by Method A was immobilized mentioned above.

Furthermore, based on FIG. 14, it can be known that as the dilution ratio of proteinase K increases, the stripping degree of the magnetic bead from the test strip containing magnetic beads on which $\alpha$1-23-mer-NH$_2$ peptide prepared by Method B was immobilized relatively decreases, and that represents that the stripping of the magnetic beads is caused by proteinase K. Similarly, based on FIG. 15, it can be known that as the dilution ratio of trypsin increases, the stripping degree of the magnetic bead from the test strip containing magnetic beads on which $\beta$2-23-mer-NH$_2$ peptide prepared by Method B was immobilized relatively decreases, and that represents that the stripping of the magnetic beads is caused by trypsin.

Example 8: Lactic acid bacteria test at low temperature for test strip for microorganism detection containing magnetic beads on which peptides with modification are immobilized

At 4°C (temperature for simulating the storage ambient of vacuum-packed meat), the test strips obtained by item 3.1.2 of Example 3 (containing magnetic beads on which $\alpha$1-23-mer-NH$_2$ peptide or $\beta$2-23-mer-NH$_2$ peptide was immobilized) were tested by different lactic acid bacteria. M.R.S. medium was used in the control group.

The test strip was co-cultured with lactic acid bacteria in a 4°C refrigerator for 2 days. The strip was taken out and photographed every day and image analysis was performed by ImageJ. Three parameters, digestion level (DL) of peptide, area change (AC) of the region covered by the magnetic beads and detachment level, were calculated. Calculation formula for digestion level of peptide, area change (AC) of the region covered by the magnetic beads, and detachment level are shown as above.

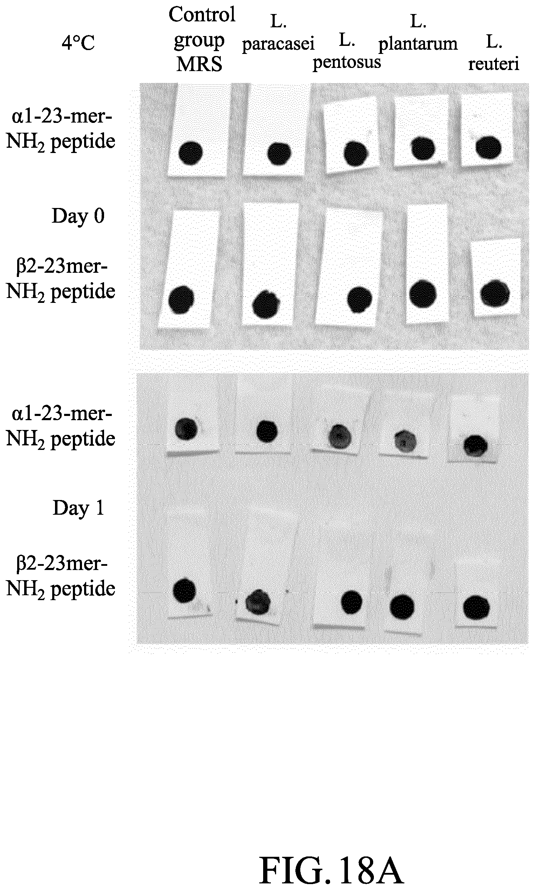

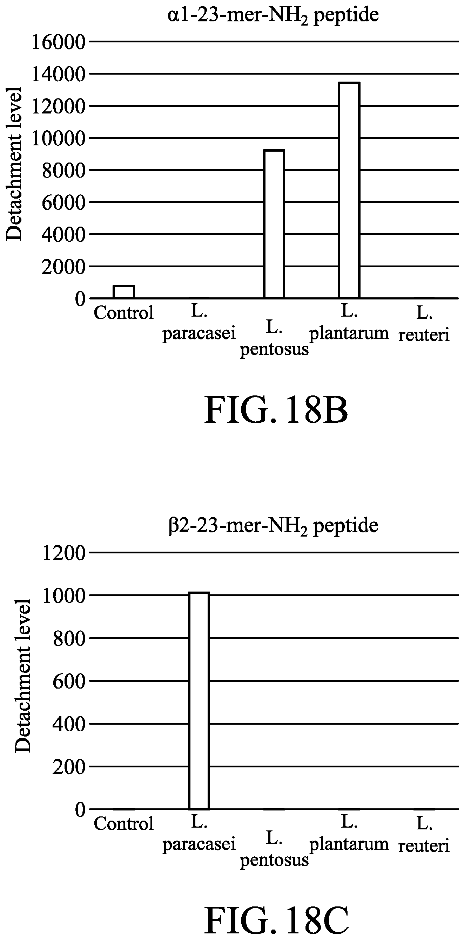

The results are as shown in FIGs. 18A to 18C.

FIGs. 18A to 18C show test results for the test strips containing magnetic beads on which $\alpha$1-23-mer-NH$_2$ peptide or $\beta$2-23-mer-NH$_2$ peptide was immobilized.

According to FIGs. 18A to 18C, it can be known that the test strip of the present disclosure can detect lactic acid bacteria at a low temperature, and different lactic acid bacteria have their preference, in which the $\alpha$1-23-mer-NH$_2$ peptide can be decomposed by more kinds of lactic acid bacteria. For the test strip containing magnetic beads on which $\alpha$1-23-mer-NH$_2$ peptide was immobilized, on Day 1 in the low-temperature culturing, it could react with *Lactobacillus plantarum* and *Lactobacillus pentosus,* and the stripping of the magnetic beads could be observed, and on Day 2, except that no obvious stripping of the magnetic beads was observed in the co-culture group with *Lactobacillus paracasei,* the stripping of the magnetic beads was observed in the co-culture groups with other three strains *(Lactobacillus plantarum, Lactobacillus pentosus* and *Lactobacillus reuteri*), and that represented that the test strip was capable of detecting the bacterial species with which it was co-cultured. As for the test strip containing magnetic beads on which $\beta$2-23-mer-NH$_2$ peptide was immobilized, it could react with *Lactobacillus paracasei* on Day 2.

Example 9

Confirmation of the minimum detectable amount of lactic acid bacteria for test strip

The test strips containing magnetic beads on which $\alpha$1-23-mer-NH$_2$ peptide obtained by item 3.1.2 of Example 3 was immobilized was test by *Lactobacillus plantarum.* Positive control: proteinase K; negative control: MRS medium.

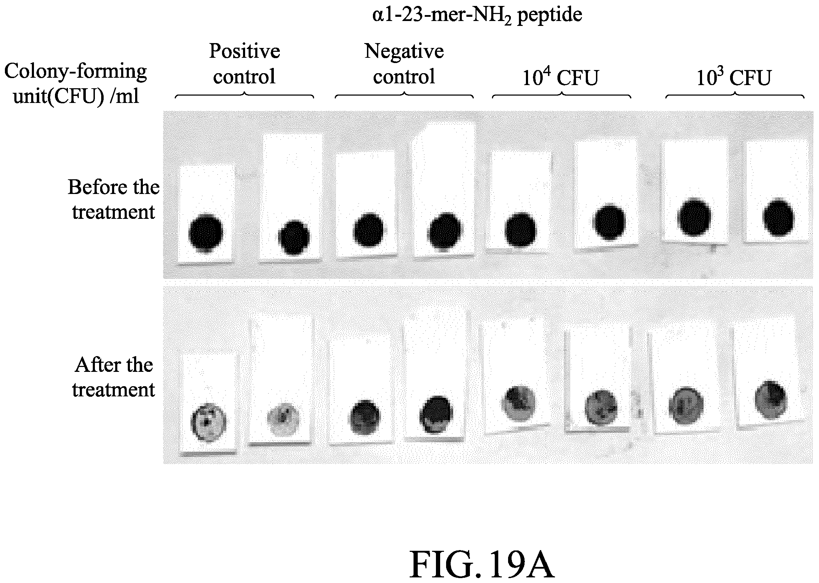

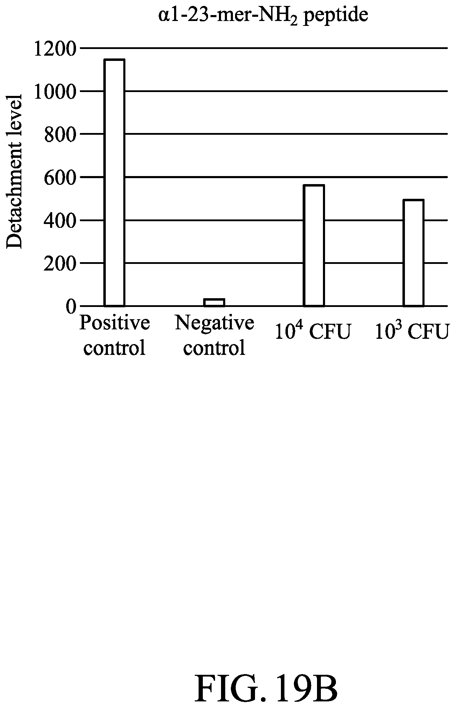

$10^3$ or $10^4$ colony-forming unit (cfu) of bacteria was co-cultured with the test strip at 4°C for 18 hours. The results are as shown in FIGs. 19A and 19B.

FIG. 19A shows a photograph for co-culturing the test strips containing magnetic beads on which $\alpha$1-23-mer-NH$_2$ peptide was immobilized with the *Lactobacillus plantarum* with different amount of bacteria in a confirmation test for the minimum detectable amount of *Lactobacillus plantarum* for the test strip. FIG. 19B shows the detachment level of the test strips containing magnetic beads on which $\alpha$1-23-mer-NH$_2$ peptide was immobilized in a confirmation test for the minimum detectable amount of *Lactobacillus plantarum* for the test strip.

According to FIGs. 19A and 19B, it can be known that the test strip can detect bacteria in the group with the initial bacterial amount of $10^3$ and $10^4$ colony forming units.

According to the above results, it can be known that the test strip of the present disclosure can effectively detect the target bacteria at a low bacterial amount ($10^3$ colony forming units), and thus the test strip of the present disclosure has high sensitivity and can be effectively applied to detection of microorganisms in food, and can be used for microorganism detection at low temperatures.

It will be apparent to those skilled in the art that various modifications and variations can be made to the disclosed embodiments. It is intended that the specification and examples be considered as exemplary only, with a true scope of the disclosure being indicated by the following claims.

**Claims**

1. A test strip for microorganism detection, comprising:

    a porous substrate, a surface of which has a first color; and
    a microorganism detection composition which is at least adhered to a part of a surface of the porous substrate or at least adhered to a part of a surface of the porous substrate and a part of an interior of the porous substrate, comprising:

    a plurality of colored particles having a second color that is different than the first color; and
    a plurality of specific peptides immobilized on the surfaces of the plurality of colored particles, in which the plurality of specific peptides connect the plurality of colored particles to each other to make the plurality of colored particles aggregate to cover the part of the surface of the porous substrate and present the second color on the part of the surface of the porous substrate,

    wherein the specific peptide is designed for a specific microorganism, and the specific microorganism has an ability to cleave the specific peptide.

2. The test strip for microorganism detection as claimed in claim 1, wherein the surface of the colored particle is modified with at least one functional group.

3. The test strip for microorganism detection as claimed in claim 2, wherein the at least one functional group comprises -COOH, -NH$_2$ or -SH.

4. The test strip for microorganism detection as claimed in any one of claims 1-3, wherein the length of the specific peptide is 10-200 amino acids.

5. The test strip for microorganism detection as claimed in any one of claims 1-4, wherein the specific microorganism is a lactic acid bacterium.

6. The test strip for microorganism detection as claimed in claim 5, wherein the sequence of the specific peptide comprises a sequence having at least about 80% sequence identity to the amino acid sequence of SEQ ID NO. 1, SEQ ID NO. 2, SEQ ID NO. 3, SEQ ID NO. 4 or SEQ ID NO. 5.

7. The test strip for microorganism detection as claimed in claim 6, wherein the sequence of the specific peptide is the amino acid sequence of SEQ ID NO. 1 or SEQ ID NO. 6.

8. The test strip for microorganism detection as claimed in claim 6, wherein the sequence of the specific peptide is the amino acid sequence of SEQ ID NO. 2 or SEQ ID NO. 7.

9. A microorganism detection method, comprising:

bringing a test sample into contact with the test strip for microorganism detection as claimed in claim 1; and determining the color change of the test strip for microorganism detection after the test sample comes into contact with the test strip for microorganism detection,
wherein when the test sample contains the specific microorganism, the specific microorganism cleaves the specific peptide, and that leads to the plurality of colored particles no longer aggregating to lighten the second color, which is present due to the aggregation of the plurality of colored particles, and/or to expose part of the surface of the porous substrate, thereby making the first color visible.

10. The microorganism detection method as claimed in claim 9, wherein the microorganism comprises a bacterium or a fungi.

11. The microorganism detection method as claimed in claim 10, wherein the bacterium comprises a lactic acid bacterium, *Escherichia coli,* a bacterium belonging to the genus *Salmonella, Staphylococcus aureus,* a bacterium belonging to the genus *Listeria* or a bacterium belonging to the genus *Shigella.*

12. The microorganism detection method as claimed in claim 11, wherein the bacterium is a lactic acid bacterium, and the lactic acid bacterium comprises *Lactobacillus reuteri, Lactobacillus pentosus, Lactobacillus gasseri, Lactobacillus salivarius, Lactobacillus paracasei* and/or *Lactobacillus plantarum.*

13. The microorganism detection method as claimed in any one of claims 9-12, wherein the sequence of the specific peptide comprises a sequence having at least about 80% sequence identity to the amino acid sequence of SEQ ID NO. 1, SEQ ID NO. 2, SEQ ID NO. 3, SEQ ID NO. 4 or SEQ ID NO. 5.

14. The microorganism detection method as claimed in claim 13, wherein the sequence of the specific peptide is the amino acid sequence of SEQ ID NO. 1 or SEQ ID NO. 6.

15. The microorganism detection method as claimed in claim 13, wherein the sequence of the specific peptide is the amino acid sequence of SEQ ID NO. 2 or SEQ ID NO. 7.

**Patentansprüche**

1. Teststreifen zum Nachweis von Mikroorganismen, mit:

einem porösen Substrat, dessen Oberfläche eine erste Farbe aufweist; und
einer Zusammensetzung zum Nachweis von Mikroorganismen, die zumindest an einem Teil einer Oberfläche des porösen Substrats anhaftet oder zumindest an einem Teil einer Oberfläche des porösen Substrats und an einem Teil eines Inneren des porösen Substrats anhaftet, mit:

mehreren farbigen Partikeln mit einer zweiten Farbe, die sich von der ersten Farbe unterscheidet; und
mehreren spezifischen Peptiden, die auf den Oberflächen der mehreren farbigen Partikel immobilisiert sind, wobei die mehreren spezifischen Peptide die mehreren farbigen Partikeln miteinander verbinden, um zu veranlassen, dass die mehreren farbigen Partikeln aggregieren, so dass sie den Teil der Oberfläche des porösen Substrats bedecken und die zweite Farbe auf dem Teil der Oberfläche des porösen Substrats präsentieren,

wobei das spezifische Peptid für einen spezifischen Mikroorganismus designt ist und der spezifische Mikroorganismus in der Lage ist, das spezifische Peptid zu spalten.

2. Teststreifen nach Anspruch 1, wobei die Oberfläche des farbigen Partikels mit mindestens einer funktionellen Gruppe modifiziert ist.

3. Teststreifen nach Anspruch 2, wobei mindestens eine funktionelle Gruppe -COOH, -NH$_2$ oder -SH enthält.

4. Teststreifen nach einem der Ansprüche 1 bis 3, wobei die Länge des spezifischen Peptids 10 bis 200 Aminosäuren beträgt.

5. Teststreifen nach einem der Ansprüche 1 bis 4, wobei der spezifische Mikroorganismus ein Milchsäurebakterium ist.

6. Teststreifen nach Anspruch 5, wobei die Sequenz des spezifischen Peptids eine Sequenz mit mindestens etwa 80% Sequenzidentität zur Aminosäuresequenz von SEQ ID NR. 1, SEQ ID NR. 2, SEQ ID NR. 3, SEQ ID NR. 4 oder SEQ ID NR. 5 aufweist.

7. Teststreifen nach Anspruch 6, wobei die Sequenz des spezifischen Peptids die Aminosäuresequenz von SEQ ID NR. 1 oder SEQ ID NR. 6 ist.

8. Teststreifen nach Anspruch 6, wobei die Sequenz des spezifischen Peptids die Aminosäuresequenz von SEQ ID NR. 2 oder SEQ ID NR. 7 ist.

9. Verfahren zum Nachweis von Mikroorganismen, mit den Schritten:

Inkontaktbringen einer Testprobe mit dem Teststreifen zum Nachweis von Mikroorganismen nach Anspruch 1; und
Bestimmen der Farbänderung des Teststreifens zum Nachweis von Mikroorganismen, nachdem die Testprobe mit dem Teststreifen zum Nachweis von Mikroorganismen in Kontakt gebracht worden ist,
wobei, wenn die Testprobe den spezifischen Mikroorganismus enthält, der spezifische Mikroorganismus das spezifische Peptid spaltet, was dazu führt, dass die mehreren farbigen Partikel nicht mehr aggregieren, um die zweite Farbe zu intensivieren, die aufgrund der Aggregation der mehreren farbigen Partikel vorhanden ist, und/oder um einen Teil der Oberfläche des porösen Substrats freizulegen, wodurch die erste Farbe sichtbar wird.

10. Verfahren nach Anspruch 9, wobei der Mikroorganismus ein Bakterium oder einen Pilz aufweist.

11. Verfahren nach Anspruch 10, wobei das Bakterium ein Milchsäurebakterium, Escherichia coli, ein Bakterium der Gattung Salmonella, Staphylococcus aureus, ein Bakterium der Gattung Listeria oder ein Bakterium der Gattung Shigella aufweist.

12. Verfahren nach Anspruch 11, wobei das Bakterium ein Milchsäurebakterium ist und das Milchsäurebakterium Lactobacillus reuteri, Lactobacillus pentosus, Lactobacillus gasseri, Lactobacillus salivarius, Lactobacillus paracasei und/oder Lactobacillus plantarum aufweist.

13. Verfahren nach einem der Ansprüche 9 bis 12, wobei die Sequenz des spezifischen Peptids eine Sequenz mit mindestens etwa 80% Sequenzidentität zu der Aminosäuresequenz von SEQ ID NR. 1, SEQ ID NR. 2, SEQ ID NR. 3, SEQ ID NR. 4 oder SEQ ID NR. 5 aufweist.

14. Verfahren nach Anspruch 13, wobei die Sequenz des spezifischen Peptids die Aminosäuresequenz von SEQ ID NR. 1 oder SEQ ID NR. 6 ist.

15. Verfahren nach Anspruch 13, wobei die Sequenz des spezifischen Peptids die Aminosäuresequenz von SEQ ID NR. 2 oder SEQ ID NR. 7 ist.

**Revendications**

1. Bandelette de test pour la détection de microorganismes, comprenant :

   un substrat poreux, dont une surface possède une première couleur ; et
   une composition pour la détection de microorganismes qui a adhéré au moins sur une partie de surface du substrat poreux ou a adhéré au moins sur une partie de surface du substrat poreux et une partie d'intérieur du substrat poreux, comprenant :

   une pluralité de particules colorées ayant une deuxième couleur qui est différente de la première couleur ; et
   une pluralité de peptides spécifiques immobilisés sur les surfaces de la pluralité des particules colorées, où les peptides spécifiques de la pluralité se lient mutuellement aux particules colorées de la pluralité pour former la pluralité d'agrégats de particules colorées afin de recouvrir la partie de la surface du substrat poreux et présenter la deuxième couleur sur la partie de la surface du substrat poreux, où le peptide spécifique est prévu pour un microorganisme spécifique, et le microorganisme spécifique a la capacité de réaliser une scission du peptide spécifique.

2. Bandelette de test pour la détection de microorganismes selon la revendication 1, dans laquelle la surface de la particule colorée est modifiée avec au moins un groupe fonctionnel.

3. Bandelette de test pour la détection de microorganismes selon la revendication 2, dans laquelle l'au moins un groupe fonctionnel comprend -COOH, -NH$_2$ ou -SH.

4. Bandelette de test pour la détection de microorganismes selon l'une quelconque des revendications 1 à 3, dans laquelle la longueur du peptide spécifique est de 10 à 200 acides aminés.

5. Bandelette de test pour la détection de microorganismes selon l'une quelconque des revendications 1 à 4, dans laquelle le microorganisme spécifique est une bactérie lactique.

6. Bandelette de test pour la détection de microorganismes selon la revendication 5, dans laquelle la séquence du peptide spécifique comprend une séquence ayant au moins 80 % d'identité de séquence avec la séquence d'acides aminés de la SEQ ID N° 1, de la SEQ ID N° 2, de la SEQ ID N° 3, de la SEQ ID N° 4, ou de la SEQ ID N° 5.

7. Bandelette de test pour la détection de microorganismes selon la revendication 6, dans laquelle la séquence du peptide spécifique est une séquence d'acides aminés de la SEQ ID N° 1 ou de la SEQ ID N° 6.

8. Bandelette de test pour la détection de microorganismes selon la revendication 6, dans laquelle la séquence du peptide spécifique est une séquence d'acides aminés de la SEQ ID N° 2 ou de la SEQ ID N° 7.

9. Procédé de détection de microorganismes, comprenant :

   la mise en contact d'un échantillon de test avec la bandelette de test pour la détection de microorganismes selon la revendication 1 ; et
   la détermination du changement de couleur de la bandelette de test pour la détection de microorganismes après que l'échantillon de test ait été mis en contact avec la bandelette de test pour la détection de microorganismes, où, lorsque l'échantillon de test contient le microorganisme spécifique, le microorganisme spécifique réalise une scission du peptide spécifique, et ceci mène à la pluralité des particules colorées ne s'agrégeant plus pour éclaircir la deuxième couleur, qui est présente due à l'agrégation de la pluralité des particules colorées, et/ou pour exposer une partie de la surface du substrat poreux, rendant ainsi la première couleur visible.

10. Procédé de détection de microorganismes selon la revendication 9, dans lequel le microorganisme comprend une bactérie ou des champignons.

11. Procédé de détection de microorganismes selon la revendication 10, dans lequel la bactérie comprend une bactérie lactique, l'*Escherichia coli,* une bactérie faisant partie du gène de la *Salmonella,* du *Staphylococcus aureus,* une bactérie faisant partie du gène de la *Listeria* ou une bactérie faisant partie du gène de la *Shigella.*

12. Procédé de détection de microorganismes selon la revendication 11, dans lequel la bactérie est une bactérie lactique, et la bactérie lactique comprend le *Lactobacillus reuteri,* le *Lactobacillus pentosus,* le *Lactobacillus gasseri,* le *Lactobacillus salivarius,* le *Lactobacillus paracasei* et/ou le *Lactobacillus plantarum.*

**13.** Procédé de détection de microorganismes selon l'une quelconque des revendications 9 à 12, dans lequel la séquence du peptide spécifique comprend une séquence ayant au moins 80 % d'identité de séquence avec la séquence d'acides aminés de la SEQ ID N° 1, de la SEQ ID N° 2, de la SEQ ID N° 3, de la SEQ ID N° 4, ou de la SEQ ID N° 5.

**14.** Procédé de détection de microorganismes selon la revendication 13, dans lequel la séquence du peptide spécifique est une séquence d'acides aminés de la SEQ ID N° 1 ou de la SEQ ID N° 6.

**15.** Procédé de détection de microorganismes selon la revendication 13, dans lequel la séquence du peptide spécifique est une séquence d'acides aminés de la SEQ ID N° 2 ou de la SEQ ID N° 7.

FIG. 1A

EP 3 502 268 B1

100

## FIG. 1B

100

## FIG. 1C

FIG. 2

FIG. 3

FIG.4A

FIG. 4B

FIG. 4C

## FIG. 5

EP 3 502 268 B1

Photocopy paper — Filter paper — Nitrocellulose membrane — PVDF membrane

FIG. 6

EP 3 502 268 B1

20000X

10000X

FIG. 7

Proteinase
K

α1-23-
mer peptide

Before the treatment

Control   1X    2X    10X   $10^2$X   $10^3$X   $10^4$X

After the treatment

Control 1X    2X    10X   $10^2$X  $10^3$X   $10^4$X

## FIG. 8A

α1-23-mer peptide

## FIG. 8B

Before the treatment                    After the treatment

Trypsin  Control  1X    2X    10X   20X   200X 1000X    Control 1X    2X    10X   20X   200X 1000X

β2-23-
mer peptide

## FIG. 9A

β2-23-mer peptide

## FIG. 9B

FIG. 10A

FIG. 10B

## FIG.11A

β2-23-mer peptide

Control
group PBS L. reuteri | L. pentosus | L. gasseri | L. salivarius | L. plantarum | L. paracasei

Before the treatment

After the treatment

## FIG.11B

β2-23-mer peptide

Detachment level

Control group | L. reuteri | L. pentosus | L. gasseri | L. salivarius | L. plantarum | L. paracasei

5000 4500 4000 3500 3000 2500 2000 1500 1000 500 0

α1-23-mer-NH$_2$ peptide

FIG. 12A

α1-23-mer-NH$_2$ peptide

FIG. 12B

α1-23-mer-NH$_2$ peptide

FIG. 12C

α1-23-mer-NH$_2$ peptide

FIG. 12D

β2-23-mer-NH$_2$ peptide

FIG. 13A

β2-23-mer-NH$_2$ peptide

FIG. 13B

β2-23-mer-NH$_2$ peptide

FIG. 13C

β2-23-mer-NH$_2$ peptide

FIG. 13D

FIG. 14

FIG. 15

α1-35-mer-NH₂ peptide
Dilution ratio of proteinase K

FIG. 16A

FIG. 16B

β2-35-mer-NH$_2$ peptide
Dilution ratio of Trypsin

FIG. 17A

FIG. 17B

FIG.18A

α1-23-mer-NH$_2$ peptide

FIG. 18B

β2-23-mer-NH$_2$ peptide

FIG. 18C

FIG. 19A

FIG. 19B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62608177 B

**Non-patent literature cited in the description**

- **L. ZHAO et al.** *ACS APPLIED MATERERIALS & INTERFACES,* 18 February 2015, vol. 7, 5177-5186

SEQUENCE LISTING

<110> Tseng-Huang LIU Chia-Ying TANG Pei-Shin JIANG

<120> TEST STRIP FOR MICROORGANISM DETECTION AND DETECTION METHOD USING THE SAME

<130> 0965-A25575-US

<150> US 62/608,177
<151> 2017-12-20

<160> 7

<170> PatentIn version 3.5

<210> 1
<211> 23
<212> PRT
<213> Artificial Sequence

<220>
<223> Artificial peptide sequence

<400> 1

```
Arg Pro Lys His Pro Ile Lys His Gln Gly Leu Pro Gln Glu Val Leu
1               5                   10                  15

Asn Glu Asn Leu Leu Arg Phe
            20
```

<210> 2
<211> 23
<212> PRT
<213> Artificial Sequence

<220>
<223> Artificial peptide sequence

<400> 2

```
Ile Asn Lys Lys Ile Glu Lys Phe Gln Ser Glu Glu Gln Gln Gln Thr
1               5                   10                  15

Glu Asp Glu Leu Gln Asp Lys
            20
```

<210> 3
<211> 23
<212> PRT
<213> Artificial Sequence

<220>
<223> Artificial peptide sequence

<400> 3

```
Glu Lys Phe Gln Ser Glu Glu Gln Gln Gln Thr Glu Asp Glu Leu Gln
1               5                   10                  15

Asp Lys Ile His Pro Phe Ala
                20
```

<210> 4
<211> 23
<212> PRT
<213> Artificial Sequence

<220>
<223> Artificial peptide sequence

<400> 4

```
Val Met Phe Pro Pro Gln Ser Val Leu Ser Leu Ser Gln Ser Lys Val
1               5                   10                  15

Leu Pro Val Pro Glu Lys Ala
                20
```

<210> 5
<211> 23
<212> PRT
<213> Artificial Sequence

<220>
<223> Artificial peptide sequence

<400> 5

```
Pro Ile Gln Ala Phe Leu Leu Tyr Gln Glu Pro Val Leu Gly Pro Val
1               5                   10                  15

Arg Gly Pro Phe Pro Ile Ile
                20
```

<210> 6
<211> 35
<212> PRT
<213> Artificial Sequence

<220>
<223> Artificial peptide sequence

<400> 6

```
Arg Pro Lys His Pro Ile Lys His Gln Gly Leu Pro Gln Glu Val Leu
1               5               10                  15


Asn Glu Asn Leu Leu Arg Phe Phe Val Ala Pro Gly Pro Glu Val Phe
            20                  25                  30


                        Gly Lys Glu
                            35
```

<210> 7
<211> 35
<212> PRT
<213> Artificial Sequence

<220>
<223> Artificial peptide sequence

<400> 7

```
Ile Asn Lys Lys Ile Glu Lys Phe Gln Ser Glu Glu Gln Gln Gln Thr
1               5               10                  15


Glu Asp Glu Leu Gln Asp Lys Ile His Pro Phe Ala Gln Thr Gln Ser
            20                  25                  30


Leu Val Tyr
        35
```